(11) **EP 3 072 578 B1**

(12) **EUROPEAN PATENT SPECIFICATION**

(45) Date of publication and mention
of the grant of the patent:
**29.04.2020 Bulletin 2020/18**

(51) Int Cl.:
***B01D 63/08*** *(2006.01)*     ***G01N 33/49*** *(2006.01)*
***C12M 1/00*** *(2006.01)*     *G01N 1/40* *(2006.01)*

(21) Application number: **16162056.2**

(22) Date of filing: **23.03.2016**

(54) **METHOD FOR ISOLATING OR DETECTING RARE CELL**

VERFAHREN ZUR ISOLIERUNG ODER DETEKTION EINER SELTENEN ZELLE

PROCÉDÉ PERMETTANT D'ISOLER OU DE DÉTECTER DES CELLULES RARES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.03.2015 JP 2015060034
22.03.2016 JP 2016057313**

(43) Date of publication of application:
**28.09.2016 Bulletin 2016/39**

(73) Proprietor: **ARKRAY, Inc.
Minami-ku
Kyoto-shi,
Kyoto 601-8045 (JP)**

(72) Inventor: **TAKAGI, Hidenori
Kyoto-shi, Kyoto 602-0008 (JP)**

(74) Representative: **Dehns
St. Bride's House
10 Salisbury Square
London EC4Y 8JD (GB)**

(56) References cited:
**WO-A1-2014/142754**     **DE-U1-202012 003 212**
**US-A1- 2011 244 443**     **US-A1- 2012 129 252**
**US-A1- 2012 178 097**

- **FRANK A. W. COUMANS ET AL: "Filter Characteristics Influencing Circulating Tumor Cell Enrichment from Whole Blood", PLOS ONE, vol. 8, no. 4, 23 April 2013 (2013-04-23), page e61770, XP055293733, DOI: 10.1371/journal.pone.0061770**

**Description**

BACKGROUND OF THE INVENTION

1. Field of the Invention

The present disclosure relates to a method for isolating or detecting a rare cell in a blood specimen, a method for analyzing a circulating tumor cell, a filter to be used in these methods, and a rare cell capturing apparatus.

2. Description of Related Art

Blood cell components mainly include erythrocytes, leukocytes, and platelets, and cells other than these cells may be present in the blood in very rare cases. One example of such rare cells is a circulating tumor cell (CTC). The CTC is a cell that is released from a primary tumor tissue or a metastatic tumor tissue and invades the blood. It is thought that cancer metastases are caused by cancer cells being carried to other parts in the body through blood vessels and lymphatic vessels and proliferating, and it has been reported that the number of CTCs in the blood is related to the possibility of cancer metastasis and the prognosis of cancer. Therefore, it is known that, in the research for using CTCs as indices for the diagnosis, prognosis, and the prediction or determination of therapeutic effects of cancer (particularly, metastatic cancer such as breast cancer), the number of CTCs in the blood is counted and the nucleic acid of the CTC is measured. Accordingly, various methods for isolating and concentrating the CTC in the blood have been proposed and studied (see Patent Documents 1 to 5 and Non-Patent Documents 1 to 6, for example).

Patent Document 1: JP 2013-17429A
Patent Document 2: JP 2010-530234A
Patent Document 3: Japanese Patent No. 5141919
Patent Document 4: JP 2013-42689A
Patent Document 5: JP 2011-163830A

Non-Patent Document 1: Vona G, Sabile A, Louha M, Sitruk V, Romana S, Schutze K, Capron F, Franco D, Pazzagli M, Vekemans M, Lacour B, Brechot C, Paterlini-Brechot P. Isolation by Size of Epithelial Tumor Cells: A New Method for the Immunomorphological and Molecular Characterization of Circulating Tumor Cells, Am J Pathol. 2000 Jan; 156(1):57-63
Non-Patent Document 2: Coumans FA, van Dalum G, Beck M, Terstappen LW. (2013) Filter Characteristics Influencing Circulating Tumor Cell Enrichment from Whole Blood, April | Volume 8 | Issue 4 | e61770
Non-Patent Document 3: Park S, Ang RR, Duffy SP, Bazov J, Chi KN, Black PC, Ma H (2014) Morphological differences between circulating tumor cells from prostate cancer patients and cultured prostate cancer cells, PLOSONE January 2014 | Volume 9 | Issue 1 | e85264
Non-Patent Document 4: Xu W, Mezencev R, Kim B, Wang L, McDonald J, Sulchek T. (2012) October 2012 | Volume 7 | Issue 10 | e46609 Cell Stiffness Is a Biomarker of the Metastatic Potential of Ovarian Cancer Cells
Non-Patent Document 5: Zhang W, Kai K, Choi DS, Iwamoto T, Nguyen YH, Wong H, Landis MD, Ueno NT, Chang J, Qin L. (2012) Microfluidics separation reveals the stem-cell-like deformability of tumor-initiating cells.
Non-Patent Document 6: R. Negishi et al. Development of the automated circulating tumor cell recovery system with microcavity array, Biosensors and Bioelectronics (2014).

US2012129252, us2012178097. WO2014142754 disclose filter systems for capturing cells using filters similar to the invention but not considering a ratio (major axis diameter of hole / distance of holes in minor axis direction) in the design for improving the collection rate.

SUMMARY OF THE INVENTION

In general, several tens of billions of erythrocytes and several tens of millions of leukocytes are contained in 10 ml of blood, whereas zero to several rare cells such as CTCs are contained therein, and several thousands of the rare cells are contained therein at most. These rare cells include medically important cells such as immunocytes and the above-described cells that are thought to have a relationship with cancer metastases. Therefore, it is anticipated that analysis of rare cells in blood will be performed more actively in the future, and a simpler method that causes little loss is required. There are many technical problems in the direct analysis of CTCs, and it is necessary to perform a treatment such as isolation and concentration. Therefore, the development of a technique for more efficiently isolating and concentrating CTCs from blood is expected.

There are some methods for the technique for isolating and concentrating CTCs, and the technique of isolation and concentration using an antibody and the technique of isolation and concentration using a filter have been reported (Non-Patent Document 1). Non-Patent Document 1 discloses a technique of isolation and concentration in which a filter with holes having a diameter of 8 μm is used to allow a large number of hemocytes to pass through the filter and allow the CTCs to remain on the filter. A method of isolation and concentration using a filter as in the method disclosed in Non-Patent Document 1 is a method that does not depend on the antigens on the surfaces of the CTCs unlike the method of isolation and concentration using an antibody.

Although the CTC is a cell that is very useful in medical development, it is said that there are only a few CTCs in 10 ml of blood as described above. If there are only two CTCs in 10 ml of blood, according to Poisson distribution, a detection rate of not more than 20% can be expected even if 100% of the CTCs can be completely captured during the treatment of 1 ml of blood. However, if a larger amount of blood can be treated and 8 ml of blood is treated, for example, the probability that one or more CTCs are present is close to about 90%. When the treatment amount of blood increases and the number of filters thus increases, the configuration becomes complicated. Therefore, a filter capable of appropriately treating a larger amount of blood at one time is expected.

Patent Document 2 discloses that a CTC has a larger size and a lower deformability than a hemocyte. On the other hand, CTCs encompass a cancer cell that is smaller than a hole having a diameter of 8 μm (Non-Patent Documents 1 and 2, for example) and a cancer cell having high deformability, and it is reported that these cells may be highly metastatic (Non-Patent Documents 4 and 5, for example). It is required that these cells are also isolated in order to diagnose and analyze cancer with high accuracy using the CTC.

Therefore, in one or more embodiments, the present disclosure provides a filter and a method for treating a blood specimen with which both a small rare cell and a rare cell having high deformability can be efficiently and easily captured in the case where rare cells are contained in a blood specimen.

In one or more embodiments, the present disclosure relates to a method for isolating or detecting a rare cell including filtering a blood specimen using a filter to isolate or detect a rare cell in the blood specimen, the filter including holes having a minor axis diameter of 5 μm or more and 8 μm or less (5 to 8 pm) and a major axis diameter of 40 μm or more at a hole density of 40 holes/mm² or more and 2000 holes/mm² or less (40 to 2000 holes/mm²) with the ratio (w/z) between the major axis diameter w (μm) and a gap z (μm) between the holes in the minor axis diameter direction being 7.0 or more and 130 or less (7.0 to 130).

In another one or more embodiments, the present disclosure relates to a filter including holes having a minor axis diameter of 5 μm or more and 8 μm or less (5 to 8 μm) and a major axis diameter of 40 μm or more at a hole density of 40 holes/mm² or more and 2000 holes/mm² or less (40 to 2000 holes/mm²) with the ratio (w/z) between the major axis diameter w (μm) and a gap z (μm) between the holes in the minor axis diameter direction being 7.0 or more and 130 or less (7.0 to 130).

In another one or more embodiments, the present disclosure relates to a rare cell capturing apparatus including: an inlet; an outlet; a channel with which the inlet and the outlet are in communication; and an isolating portion, wherein the isolating portion is arranged at a position corresponding to a portion of the channel, and includes the above-mentioned filter and a holding portion for holding the filter.

In one or more embodiments, with the present disclosure, both a small rare cell and a rare cell having high deformability can be efficiently and easily captured in the case where rare cells are contained in a blood specimen.

BRIEF DESCRIPTION OF THE DRAWINGS

FIGS. 1A and 1B are photographs of filters. FIG. 1A is an example of a filter (with slit-shaped holes) according to the present disclosure, and FIG. 2B is an example of a filter (with elliptical holes) according to a comparative example.
FIG. 2 shows an example of a rare cell capturing apparatus according to the present disclosure.
FIG. 3 is a graph showing results of Example 1. FIG. 3 shows results of the relationship between the minor axis diameter and the capture rate for SW620 cells, which are small cells.
FIG. 4 is a graph showing results of Example 2.
FIG. 5 is a graph showing results of Example 3.
FIG. 6 is a graph showing results of Example 4.
FIGS. 7A and 7B are graphs showing results of Example 5. FIG. 7A shows results when filtration pressure (ΔP1) in the entire system is 0.4 kPa, and FIG. 7B shows results when filtration pressure (ΔP1) in the entire system is 1.3 kPa.
FIG. 8 is a graph showing results of Example 8.
FIG. 9 is a graph showing results of Example 9.
FIG. 10 is a graph showing results of Example 10.
FIG. 11 is a graph showing results of Example 11.

FIG. 12 is a graph showing results of Example 12.

DETAILED DESCRIPTION OF THE INVENTION

The present inventor found the following problems in the methods described in Patent Documents 1 to 4 and Non-Patent Documents 1 to 5.

In the filter disclosed in Non-Patent Document 1, the hole density is not uniform, and some holes connect to each other to form holes that are larger than a cell. Therefore, there is a problem in that the cell is likely to pass through the filter, and thus a high capture rate cannot be obtained.

In Patent Document 1 and Non-Patent Document 2, an attempt is made to improve capturing by reducing the hole diameter in order to capture a small cell, but a filter with holes having a diameter of 5 $\mu$m or less is likely to become clogged. Therefore, there is a problem in that the operations become complicated due to damage to the cell caused by high pressure, hemolysis for reducing the number of hemocytes, an increase in the filtration area of the filter, and the like.

Patent Document 2 discloses that a rare cell is isolated using the difference in viscoelasticity of cells. However, under the conditions mentioned in Patent Document 2, the difference between pressures on both sides of the filtration film during isolation is as high as 20 kPa to 190 kPa. Therefore, there is a concern that the rare cell will be damaged during isolation, and there is a problem in that it is difficult to capture a rare cell having high deformability.

Patent Document 3 discloses that a slit filter provided with a tapered portion, a narrow portion, and a reversed tapered portion is used to capture the CTC.

Patent Document 4 and Non-Patent Document 5 describe isolation and concentration of the rare cell using a filter with slit-shaped holes. However, there is a problem in that with the filters mentioned in Patent Document 4 and Non-Patent Document 5, it is difficult to capture both a cell having high deformability and a small cancer cell when a large amount of blood is treated. In particular, with the filter according to the examples in Patent Document 4, the capture rates for a small cancer cell and a cancer cell having high deformability decreases when 8 to 10 ml of blood is treated, and thus there is a problem in that a large amount of blood cannot be treated.

The present disclosure is based on the findings that when the diameter of holes of a filter is reduced, it is difficult to capture the cell having high deformability, whereas when the hole diameter is increased in accordance with the capture rate for the cell having high deformability, the small cell is likely to pass through the filter, and that it is difficult to capture both the small cancer cell and the cancer cell having high deformability using a filter with perfectly circular holes. The present disclosure is based on the findings that in one or more embodiments, both the small rare cell and the rare cell having high deformability, which can be contained in a blood specimen, can be efficiently and easily captured by filtering the blood specimen using a filter including holes having a minor axis diameter of 5 $\mu$m or more and 8 $\mu$m or less and a major axis diameter of 40 $\mu$m or more at a hole density of 40 holes/mm$^2$ or more and 2000 holes/mm$^2$ or less with the ratio (w/z) between the major axis diameter w ($\mu$m) and the gap z ($\mu$m) between the holes in the minor axis diameter direction being 7.0 or more and 130 or less. The present disclosure is based on the findings that in one or more embodiments, by filtering a blood specimen using the above-mentioned filter, the filtration pressure condition (the filtration pressure in the entire system or the difference between pressures on the upper surface and the lower surface of the filter during the filtering) can be lowered during the filtering, as a result of which the capture rate for the rare cell having high deformability can be further increased, damage to the cell during the capturing of the cell can be preferably reduced, and the viable rare cell can be collected. The present disclosure is based on the findings that in one or more embodiments, by filtering a blood specimen using the above-mentioned filter, particularly in the case where the filtering capacity per hole of the filter is large, for example, the variation in the capture rate for the rare cell due to the difference between specimens can be reduced, and the rare cell can be preferably captured at a high capture rate compared with a filter with elliptical holes or perfectly circular holes, even when a specimen having a high hematocrit or a high concentration of leukocytes is used. The present disclosure is based on the findings that in one or more embodiments, although the capture rate for the rare cell decreases when a large amount of blood, namely about 8 to 10 ml of blood, is treated, the capture rate for the rare cell having high deformability can be further improved by filtering a blood specimen using the above-mentioned filter according to the present disclosure such that the filtering capacity per hole of the filter is 0.1 nl/$\mu$m$^2$ or more and 3 nl/$\mu$m$^2$ or less (0.1 to 3 nl/$\mu$m$^2$) in terms of the treatment capacity per hole area of the filter. As referred to herein, the "treatment capacity" is the amount of the blood specimen that can be filtered by the hole.

Since the method according to the present disclosure uses a filter, in one or more embodiments, it is easy to stain and wash a cell captured by the filter, or it is easy to observe the cell captured by the filter, for the purpose of identifying the cell as a hemocyte component, a CTC, or the like. In the case where the cells on the filter are stained, washed, and observed, the larger a filtration area is, the more complicated the operations are because the reagent amount, the washing amount, and the observation range increase. Therefore, a filter having a reduced filtration area or a filter housing having a reduced capacity is desired. With the method according to the present disclosure, in one or more embodiments, the filtration area of a filter with respect to the treatment amount of blood can be reduced.

[Rare cell]

In the present disclosure, a "rare cell" that can be contained in blood or a blood specimen is a cell other than cell components (an erythrocyte, a leukocyte, and a platelet) that can be contained in blood of a human or an animal other than a human. In one or more embodiments, examples of the rare cell include a tumor cell and/or a cancer cell. A tumor cell or a cancer cell that circulates in blood is generally called a CTC. Depending on the specimen, the number of rare cells in 10 ml of blood may be several to several tens (e.g. 5-100), and several hundreds to several thousands (e.g. 200-10,000) at most. In one or more embodiments, the "rare cell" is a cell selected from the group consisting of a cancer cell, a circulating tumor cell, a vascular endothelial cell, a vascular endothelial precursor cell, a cancer stem cell, an epithelial cell, a hematopoietic stem cell, a mesenchymal stem cell, a fetal cell, a stem cell, and combinations thereof.

[Small rare cell]

In the present disclosure, a "small rare cell" is a rare cell having a small diameter. In one or more embodiments, one example thereof is a rare cell having a size which allows the cell to pass through a perfectly circular hole having a diameter of about 10 $\mu$m.

[Rare cell having high deformability]

In the present disclosure, a "rare cell having high deformability" is a cell that has a cell size of 10 $\mu$m or more and that can pass through a circular hole having a diameter of 5 $\mu$m or more and 6.5 $\mu$m or less (5 to 6.5 pm). In one or more embodiments, one example of a rare cell having high deformability is a rare cell that is more likely to pass through the hole (that is, the ratio of the cells passing through the hole is larger) compared to a rare cell having a similar size in the case where the whole blood is fed to a filter with perfectly circular holes having a diameter of 5 $\mu$m or more and 6.5 $\mu$m or less (5 to 6.5 pm) with a treatment amount of the blood specimen per hole of 14 nl or more at 5 nl/min or more and 20 nl/min or less (5 to 20 nl/min) per hole. In one or more embodiments, one example of a cancer cell having high deformability is a SNU-1 cell.

[Blood specimen]

In the present disclosure, a "blood specimen" means a sample that can be used in the treatment method according to the present disclosure and that contains the components constituting the blood, and examples thereof include blood, a blood derived substance containing an erythrocyte component, a body fluid or urine containing blood or blood derived substance, and samples prepared therefrom. One example of blood is the blood collected from a living organism, and examples of the living organism include a human or an animal (e.g., a mammal) other than a human. Examples of the blood derived substance containing an erythrocyte component include a substance that is isolated or prepared from blood and contains an erythrocyte component, or a dilution/concentrate thereof. For example, the blood derived substance includes a hemocyte fraction obtained by removing blood plasma, a hemocyte concentrate, a substance obtained by freeze-drying blood or hemocytes, a sample obtained by hemolyzing the whole blood and removing the erythrocyte component or a hemolyzed sample, centrifuged blood, spontaneously precipitated blood, washed hemocytes, a specific fraction, and the like. In one or more embodiments that are not limited, from the viewpoint of a simple and rapid treatment and the suppression of damage to the rare cell in the blood, the blood or hemocytes that are derived from the blood and contain the hemocyte components out of these is preferable as the above-mentioned sample containing blood.

In the present disclosure, the volume of a blood specimen that passes through one hole of the filter is determined using blood collected from a human body, that is, blood containing 1000 to 20000 leukocyte cells per microliter, as a reference. Therefore, when a diluted blood specimen is used, it is easy to convert the volume of the diluted blood specimen into the volume of the blood specimen before dilution based on the number of leukocytes. Specifically, when a 10-fold diluted blood specimen is treated, treating the volume of "X" $\mu$l/hole of the diluted blood specimen corresponds to treating the volume of "X/10" $\mu$l/hole of the blood specimen before dilution.

[Method for treating blood specimen]

An aspect of the present disclosure relates to a method for filtering a blood specimen (referred to also as "treatment method according to the present disclosure" hereinafter).

<Filter>

In one or more embodiments, the filter used in filtering in the treatment method according to the present disclosure is a slit filter with a plurality of through pores (holes) having a minor axis diameter of 5 $\mu$m or more and 8 $\mu$m or less (5 to 8 $\mu$m) and a major axis diameter of 40 $\mu$m or more. In one or more embodiments, it is preferable that the through pores formed in the filter have a substantially uniform size, and it is more preferable that the through pores have substantially the same minor axis diameter and major axis diameter. In one or more embodiments, "the through pores have substantially the same minor axis diameter" means that the minor axis diameters of the through pores of the filter are in a range of the average value of the minor axis diameters $\pm 1$ $\mu$m, $\pm 0.5$ $\mu$m, $\pm 0.4$ $\mu$m, $\pm 0.3$ $\mu$m, or $\pm 0.2$ $\mu$m, the average value of the minor axis diameters being obtained with a method described below. Moreover, in one or more embodiments, "the through pores have substantially the same major axis diameter" means that the major axis diameters of the through holes of the filter are in a range of the average value of the major axis diameters $\pm 3$ $\mu$m, $\pm 2$ $\mu$m, $\pm 1$ $\mu$m, $\pm 0.5$ $\mu$m, $\pm 0.3$ $\mu$m, or $\pm 0.2$ $\mu$m, the average value of the major axis diameters being obtained in the same manner as described above.

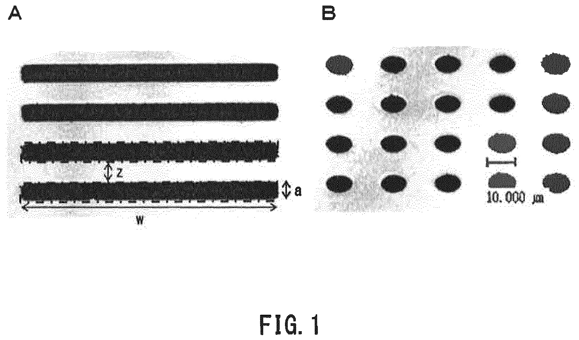

In one or more embodiments, from the viewpoint of improving the capture rate for the small rare cell, the minor axis diameter is 5 $\mu$m or more, more than 5 $\mu$m, 5.5 $\mu$m or more, 6 $\mu$m or more, or 6.5 $\mu$m or more. From the same viewpoint, the minor axis diameter is 8 $\mu$m or less, less than 8 $\mu$m, 7.5 $\mu$m or less, 7 $\mu$m or less, or 6.5 $\mu$m or less. Since the minor axis diameter is 5 $\mu$m or more, the capture rate for the rare cell having high deformability can be increased and clogging of the filter can be decreased, as a result of which the rare cell can be collected at a high capture rate. In the present disclosure, the "minor axis diameter" is the short side of a rectangle that is circumscribed about the hole (see FIG. 1A). The minor axis diameter can be obtained by using a commercially available optical microscope such as a metallographic microscope and a laser microscope, or an electron microscope to perform measurements (at 50 or more points) with a known method using software packaged with the above-mentioned microscope or commercially available image analysis software, and determining the average value, for example. Specifically, the minor axis diameter can be determined with a method described in the Examples.

In one or more embodiments, from the viewpoint of improving the capture rate for the rare cell having high deformability, the major axis diameter is 40 $\mu$m or more, 50 $\mu$m or more, 60 $\mu$m or more, 70 $\mu$m or more, or 80 $\mu$m or more. From the viewpoint of suppressing bending, distortion, and the like of the filter to improve the capture rate for the rare cell, the major axis diameter is 5000 $\mu$m or less, 4000 $\mu$m or less, 3000 $\mu$m or less, 2000 $\mu$m or less, 1000 $\mu$m or less, 970 $\mu$m or less, 900 $\mu$m or less, 800 $\mu$m or less, 700 $\mu$m or less, 600 $\mu$m or less, 500 $\mu$m or less, 400 $\mu$m or less, 300 $\mu$m or less, 200 $\mu$m or less, 150 $\mu$m or less, 120 $\mu$m or less, 100$\mu$m or less, 90 $\mu$m or less, or 80 $\mu$m or less. In the present disclosure, the "major axis diameter" is the long side of a rectangle that is circumscribed about the hole (see FIG. 1A). The major axis diameter can be obtained by using a commercially available optical microscope such as a metallographic microscope and a laser microscope, or an electron microscope to perform measurements (at 50 or more points) with a known method using software packaged with the above-mentioned microscope or commercially available image analysis software, and determining the average value, for example. Specifically, the major axis diameter can be determined with a method described in the Examples.

The filter used for filtering in the treatment method according to the present disclosure includes holes having the above-mentioned minor axis diameter and major axis diameter at a hole density of 40 holes/mm$^2$ or more and 2000 holes/mm$^2$ or less. From the viewpoint of further improving the capture rate for the rare cell having high deformability, and the viewpoint of reducing the filtration area, the hole density (the number of holes per square millimeter) is 45 holes/mm$^2$ or more, 60 holes/mm$^2$ or more, 70 holes/mm$^2$ or more, 90 holes/mm$^2$ or more, 200 holes/mm$^2$ or more, 300 holes/mm$^2$ or more, 400 holes/mm$^2$ or more, 500 holes/mm$^2$ or more, 600 holes/mm$^2$ or more, or 700 holes/mm$^2$ or more. From the same viewpoints, the hole density is 1500 holes/mm$^2$ or less, 1000 holes/mm$^2$ or less, 900 holes/mm$^2$ or less, or 800 holes/mm$^2$ or less. The hole density can be measured with a known method. For example, an image is taken using a commercially available optical microscope equipped with a 10-fold to 100-fold objective lens, and then the hole density can be calculated from the image using software packaged with the above-mentioned microscope or commercially available image analysis software.

The total number of holes included in the filtration surface of the filter can be determined by dividing the filtration area by the lengths of pitches between the centers of the holes. In addition, the total number of holes included in the filtration surface of the filter can be determined by multiplying the hole density by the filtration area. Specifically, the total number of holes can be determined with a method described in the Examples.

In the filter used for filtering in the treatment method according to the present disclosure, the holes having the above-mentioned minor axis diameter and major axis diameter are formed such that the ratio (w/z) between the major axis diameter w ($\mu$m) and the gap z ($\mu$m) between the holes in the minor axis diameter direction is 7.0 or more and 130 or less (7.0 to 130). From the viewpoint of improving the capture rates for both the small rare cell and the rare cell having high deformability, and the viewpoint of reducing variation depending on the specimen, the w/z is 7.3 or more, more than 7.3, 7.5 or more, 8.0 or more, 8.5 or more, 9.0 or more, or 10 or more. From the viewpoint of suppressing bending, distortion, and the like of the filter to improve the capture rate for the rare cell, the w/z is 130 or less, 129 or less, 120 or less, 100 or less, 90 or less, 80 or less, or 70 or less.

In the present disclosure, the "gap (z) between the holes in the minor axis diameter direction" is the shortest straight line that links the rectangles circumscribed about the holes adjacent to each other in the minor axis diameter direction. From the viewpoint of improving the capture rates for both the small rare cell and the rare cell having high deformability, and the viewpoint of reducing the variation depending on the specimen, the gap (z) between the holes in the minor axis diameter direction is 60 $\mu$m or less, 50 $\mu$m or less, 40 $\mu$m or less, 30 $\mu$m or less, 20 $\mu$m or less, or 10 $\mu$m or less. From the viewpoint of suppressing bending, distortion, and the like of the filter to improve the capture rate for the rare cell, the gap (z) between the holes in the minor axis diameter direction is 4 $\mu$m or more, 5 $\mu$m or more, 6 $\mu$m or more, 7 $\mu$m or more, 7.5 $\mu$m or more, 8 $\mu$m or more, 9 $\mu$m or more, or 10 $\mu$m or more. The gap (z) between the holes in the minor axis diameter direction can be obtained by analyzing and measuring an image (at 50 or more points) with a known method using software packaged with an electron microscope or a commercially available optical microscope such as a metallographic microscope and a laser microscope, or commercially available image analysis software, and determining the average value. Specifically, the gap (z) between the holes in the minor axis diameter direction can be determined with a method described in the Examples.

In one or more embodiments, from the viewpoint of further improving the capture rate for the rare cell having high deformability, and the viewpoint of reducing the filtration area, the area of one hole of the filter used for filtering is 200 $\mu$m$^2$ or more and 7000 $\mu$m$^2$ or less (200 to 7000 $\mu$m$^2$). The area of one hole is 250 $\mu$m$^2$ or more, 260 $\mu$m$^2$ or more, 300 $\mu$m$^2$ or more, 350 $\mu$m$^2$ or more, 390 $\mu$m$^2$ or more, 400 $\mu$m$^2$ or more, 500 $\mu$m$^2$ or more, or 550 $\mu$m$^2$ or more from the same viewpoints, and is 6800 $\mu$m$^2$ or less, 6500 $\mu$m$^2$ or less, 6300 $\mu$m$^2$ or less, or 6000 $\mu$m$^2$ or less from the same viewpoints.

In one or more embodiments, from the viewpoint of further improving the capture rate for the rare cell having high deformability, and the viewpoint of reducing the filtration area, the filter has an opening rate of 10% or more and 60% or less (10 to 60%). An "opening rate" defines the percentage of the surface area of the filter which forms the holes. The filter has an opening rate of 15% or more, 20% or more, 25% or more, 30% or more, 31% or more, 35% or more, or 40% or more from the same viewpoints, and an opening rate of 55% or less or 45% or less from the same viewpoints. The opening rate can be measured with a known method. For example, an image is taken using a commercially available optical microscope equipped with a 10-fold to 100-fold objective lens, and then the opening rate can be determined from the image using software packaged with the above-mentioned microscope or commercially available image analysis software.

In one or more embodiments, from the viewpoint of improving the capture rates for both the small rare cell and the rare cell having high deformability, the filter has a thickness of 1 $\mu$m or more and 100 $\mu$m or less (1 $\mu$m to 100 $\mu$m). From the viewpoint of improving the capture rate for the rare cell while reducing clogging of the filter, the thickness of the filter is 60 $\mu$m or less, 50 $\mu$m or less, 40 $\mu$m or less, 30 $\mu$m or less, 20 $\mu$m or less, 15 $\mu$m or less, or 10 $\mu$m or less. From the same viewpoints, the thickness of the filter is 2 $\mu$m or more, 3 $\mu$m or more, 4 $\mu$m or more, or 5 $\mu$m or more.

There is no particular limitation on the materials of the filter, but in one or more embodiments, examples of the main component include polycarbonate (PC), a parylene membrane filter, a plastic material having a Young's modulus of 1 GPa or more, a metal such as nickel (Ni), SUS, gold, silver, copper, aluminum, tungsten, and chromium, and a glass material. When cells on the filter are to be observed, it is desirable to select a plastic, a metal, or a glass material in accordance with pigments and fluorescent staining used during the observation, and combinations thereof may also be used.

The filter used for filtering in the treatment method according to the present disclosure can be produced by a known method for producing the filter. In one or more embodiments, the method includes etching, electroform processing, laser processing, or spatter processing. In one or more embodiments, the filter may be produced by cutting a filter of an arbitrary size from a sheet with the holes being patterned partially or wholly. In one or more embodiments, the filter may be produced by casting.

<Filtering condition>

In one or more embodiments, in the filtering in the treatment method according to the present disclosure, the rare cell such as the CTC can be captured by incorporating the above-described filter into a channel and introducing a blood specimen into the channel.

From the viewpoint of further improving the capture rate for the rare cell having high deformability, the treatment method according to the present disclosure includes filtering a blood specimen such that the filtering capacity per hole of the filter is 0.1 nl/$\mu$m$^2$ or more and 3.0 nl/$\mu$m$^2$ or less (0.1 to 3.0 nl/$\mu$m$^2$) in terms of the treatment capacity per unit area of the holes of the filter. From the viewpoint of reducing the filtration area, the filtering capacity per unit area of the holes of the filter is 0.1 nl/$\mu$m$^2$ or more or 0.2 nl/$\mu$m$^2$ or more. From the viewpoint of capturing a cancer cell having high deformability, the filtering capacity per unit area of the holes of the filter is 2.5 nl/$\mu$m$^2$ or less, 2 nl/$\mu$m$^2$ or less, 1.5 nl/$\mu$m$^2$

or less or 1 nl/$\mu$m$^2$ or less.

From the viewpoint of further improving the capture rate for the rare cell having high deformability, and the viewpoint of reducing damage to the cell captured during the filtering, the filtration pressure ($\Delta P_1$) during filtering in the treatment method according to the present disclosure is 0.1 kPa or more and 2.6 kPa or less (0.1 to 2.6 kPa). The filtration pressure is 0.1 kPa or more or 0.2 kPa or more from the same viewpoints, and is 2.0 kPa or less, 1.5 kPa or less, 1.3 kPa or less, 1.0 kPa or less, or 0.5 kPa or less from the same viewpoints. In the present disclosure, the "filtration pressure during filtering" is the pressure difference in the entire system from the entrance of the system to the exit of the system, and is the pressure difference in the system including the channel from a tank in which a blood specimen is placed to the waste liquid tank, for example. Therefore, the filtration pressure may be larger than the above-mentioned pressure values depending on the structure of the channel. The filtration pressure during filtering (pressure difference in the system) can be measured with a known method using a commercially available pressure gauge, or is calculated from the relationship between the flow amount and the structure of the channel.

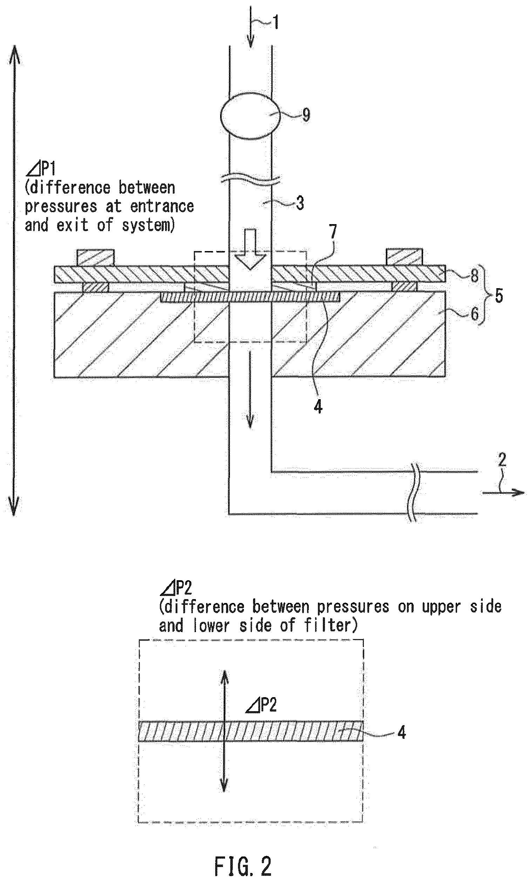

In one or more embodiments, from the viewpoint of further improving the capture rate for the rare cell having high deformability, and the viewpoint of reducing damage to the cell captured during filtering, the difference between pressures on the upper surface and the lower surface of the filter ($\Delta P_2$) during filtering in the treatment method according to the present disclosure is 100 Pa or less. In one or more embodiments, $\Delta P_2$ is 0.1 Pa or more or 1 Pa or more from the same viewpoints, and is 70 Pa or less, 50 Pa or less, 30 Pa or less, 15 Pa or less, or 10 Pa or less from the same viewpoints. In the present disclosure, the "difference between pressures on the upper surface and the lower surface of the filter ($\Delta P_2$)" is the difference between the pressure on the upper side of the filter and the pressure on the lower side of the filter (see FIG. 2). The pressure difference can be calculated from a well-known calculation equation. The pressure difference in a slit filter can be determined using the following equation:

$$\Delta P_2 = Q/N_0 \times 12\eta L/[(1-0.63)(h/w)] \times (1/h^3 w)$$

where Q is the total average value of the flow amounts of all the holes, h is the minor axis diameter, w is a major axis diameter, L is the channel length of the hole, $\eta$ is the viscosity of the liquid to be fed, and $N_0$ is the number of holes. Blood is a non-Newtonian liquid, and even when the systems have the same pressure difference, the average flow rate varies depending on the viscosity, which varies depending on the hematocrit (Hct). Therefore, the average flow rate is in a certain range. In the case where the pressure difference, $\Delta P_2$, is set when the whole blood is used, it is sufficient that the feeding and the pressure are set using $\eta$ of 4.5 mPa·S for the sake of convenience such that $\Delta P_2$ is in the above-mentioned range according to calculations. When a blood specimen has a hematocrit value (Hct) of less than 20 due to dilution or the like, it is sufficient that the feeding and the pressure are set using $\eta$ of 1 to 2 mPa·S such that $\Delta P_2$ is in the above-mentioned range according to calculations. Alternatively, when a blood specimen is diluted until the viscosity caused by blood plasma and Hct has no influence (the whole blood is diluted 4 to 10 times, for example), $\Delta P_2$ may be calculated based on the viscosity of the diluted liquid, and the diluted liquid may be fed. When feeding is performed at a constant flow amount using a syringe pump or the like, it is sufficient that the feeding is performed such that $\Delta P_2$ is in the above-mentioned range. Such a feeding condition can be achieved using a mechanism capable of performing feeding at a constant pressure, a mechanism capable of performing feeding at a constant flow amount, or the like that is well known to a person skilled in the art.

There is no particular limitation on the filtration area for filtering in the treatment method according to the present disclosure. In one or more embodiments, the filtration area can be set based on the number of holes (hole area) and the treatment amount of blood. In one or more embodiments, from the viewpoint of reducing the filtration area to reduce the amount of reagents used in the staining operation and labeling operation after filtering, the viewpoint of the observation operation, and the viewpoint of efficiently capturing the rare cell, the filtration area is 5 mm$^2$ or more and 200 mm$^2$ or less (5 mm$^2$ to 200 mm$^2$). The filtration area is 10 mm$^2$ or more from the same viewpoints, and is 150 mm$^2$ or less, 100 mm$^2$ or less, or 80 mm$^2$ or less from the same viewpoints. From the viewpoint of reducing the filtration area of the filter with respect to the treatment amount of blood, the filtration area is 100 mm$^2$ or less, 80 mm$^2$ or less, 50 mm$^2$ or less, 40 mm$^2$ or less, 30 mm$^2$ or less, or 25 mm$^2$ or less.

Although there is no particular limitation on the feeding of a blood specimen into a channel for filtering in the treatment method according to the present disclosure, it is sufficient that there is a driving force with which the blood specimen can be fed. As referred to herein "feeding" refers to delivering the blood specimen to the filter, e.g. into an apparatus. In one or more embodiments, examples of a method for introducing a blood specimen into a channel include a method of applying pressure at the entrance of the channel, a method of reducing pressure at the exit of the channel, and a method using a syringe pump or a peristaltic pump. By feeding a blood specimen at an average flow rate of 1 mm/min or more and 600 mm/min or less per hole of the filter, for example, for the purpose of introducing the blood specimen into a channel, the rare cell can be more appropriately captured compared with a conventional example. In one or more embodiments, the condition for feeding of a blood specimen is a flow rate of 2 mm/min or more and 300 mm/min or less (2 to 300 mm/min), a flow rate of 3 mm/min or more and 100 mm/min or less (3 to 100 mm/min), a flow rate of 4 mm/min or more and 80 mm/min or less (4 to 80 mm/min), or a flow rate of 4 mm/min or more and 40 mm/min or less (4 to 40 mm/min). The feeding condition may also be set in terms of the average flow amount. A method for calculating the average flow amount per hole is as follows: the average flow amount is determined by measuring the flow amount at the exit for the filtration waste liquid per unit time (total average value of the flow amounts of all the holes) or the filtration time per unit amount, and then the average flow amount is divided by the number of holes to obtain the average flow amount per hole. Moreover, the average flow rate can be determined by dividing the average flow amount by the hole area. It is sufficient that the feeding is performed such that the average flow rate per hole or the average flow amount is in the above-described range. In one or more embodiments, the average flow rate may be calculated by dividing the flow amount at the exit for the filtration waste liquid (total average value of the flow amounts of all the holes) by the total hole area.

[Method for isolating or detecting rare cell in blood specimen]

When filtering in the treatment method according to the present disclosure is performed, the rare cell (if any) remains on the filter after filtration of a blood specimen, and thus the rare cell can be isolated or detected. Therefore, another aspect of the present disclosure relates to a method for isolating or detecting a rare cell in a blood specimen including isolating or detecting a rare cell by treating a blood specimen with the treatment method according to the present disclosure.

[Labeling of rare cell in blood]

The rare cell in the blood may be labeled in the treatment method according to the present disclosure. In one or more embodiments, labeling may be performed simultaneously with isolation after filtering, or after the isolation. The labeling of the rare cell is useful in the above-described isolating method or detecting method according to the present disclosure and in a method for measuring the number of CTCs, which will be described later, and can be useful in the analysis of the isolated rare cell. Therefore, in one or more embodiments, the treatment method according to the present disclosure includes performing labeling. Moreover, the labeling of the rare cell is useful in a method for measuring the number of CTCs, which will be described later, and can be useful in the analysis of the isolated rare cell. Therefore, in one or more embodiments, the isolating method or detecting method according to the present disclosure includes performing labeling.

In one or more embodiments, the labeling can be performed by bringing a known labeling reagent into contact with the rare cell, and typically by mixing the labeling reagent with a blood specimen. In one or more embodiments, examples of the labels include a radioactive label, a fluorescent pigment label, pigment staining or a pigment label, a magnetic label, an electric-charge label, and combinations thereof, but are not limited thereto. The labeling can be performed using a labeling reagent suitable for each label.

That is, the rare cell can be labeled with a labeling method that can be used for a detecting method selected from a group consisting of a detecting method using a radioactive substance, a detecting method using luminescence, a detecting method using a pigment, a detecting method using magnetism, an electrical detecting method, an optical detecting method, and combinations thereof.

In the treatment method according to the present disclosure, the isolation or detection of the rare cell can be achieved by detecting the change in the above-mentioned filter electrically, by weight, or optically.

[Method for analyzing rare cell in blood specimen]

Another aspect of the present disclosure relates to a method for analyzing a rare cell in a blood specimen including isolating or detecting a rare cell with the isolating method or detecting method according to the present disclosure and analyzing the rare cell with a method including observing the kinetics of the cell or measuring the activity of the cell, or with a genetic method after the isolation or detection. That is, with the present disclosure, a viable cell can be captured, thus making it possible to isolate or detect a rare cell with the isolating method or detecting method according to the present disclosure and to analyze the rare cell with a method including observing the kinetics of the cell or measuring the activity of the cell, or with a genetic method after the isolation or detection. Moreover, the isolated rare cell can be collected and cultured. Alternatively, gene analysis may be performed on the target cell on the filter, or the target cell may be collected from the filter and subjected to gene analysis. Examples of the gene analysis include analysis of chromosomes and analysis of a single base mutation, and a known technique can be used to analyze genes.

[Method for measuring the number of CTCs]

It has been reported that the number of CTCs in blood is related to cancer metastases and the prognosis of cancer. Therefore, research using CTCs as indices for the diagnosis of cancer, the prognosis of cancer, and the prediction or determination of therapeutic effects is ongoing, and the number of CTCs in the blood is measured. With the treatment method according to the present disclosure and/or the isolating method or detecting method according to the present disclosure, the CTC, which is a rare cell, can be isolated while damage to the CTC is suppressed. Therefore, another aspect of the present disclosure relates to a method for measuring the number of CTCs in a blood specimen including treating the blood specimen with the treatment method according to the present disclosure to isolate or detect the rare cell, and/or isolating the CTC from the blood specimen with the isolating method or detecting method according to the present disclosure. It should be noted that the counting of the number of CTCs or the measurement of the nucleic acid of the CTC may be performed with flow cytometry simultaneously with the isolation after a cancer cell is measured from the morphological viewpoint or labeled appropriately, or may be performed by observing the isolated cells under a microscope.

[Filter]

Yet another aspect of the present disclosure relates to a filter to be used in the treatment method according to the present disclosure, the isolating method or detecting method according to the present disclosure, and/or the method for measuring the number of CTCs according to the present disclosure, the filter including holes having a minor axis diameter of 5 $\mu$m or more and 8 $\mu$m or less (5 to 8 pm) and a major axis diameter of 40 $\mu$m or more at a hole density of 40 holes/mm$^2$ or more and 2000 holes/mm$^2$ or less (40 to 2000 holes/mm$^2$) with the ratio (w/z) between the major axis diameter w ($\mu$m) and the gap z ($\mu$m) between the holes in the minor axis diameter direction being 7.0 or more and 130 or less (7.0 to 130). The holes of the filter according to the present disclosure have the same shape and the like as those of the filter used in the above-described treatment method according to the present disclosure.

[Rare cell capturing apparatus]

Yet another aspect of the present disclosure relates to a rare cell capturing apparatus for capturing a rare cell contained in a specimen. In one or more embodiments, the rare cell capturing apparatus according to the present disclosure includes an inlet, an outlet, a channel with which the inlet and the outlet are in communication, and an isolating portion. The isolating portion is arranged at a position corresponding to a portion of the channel, and includes the filter according to the present disclosure and a filter holding portion. With the rare cell capturing apparatus according to the present disclosure, the method for treating a blood specimen according to the present disclosure can be performed using the filter according to the present disclosure.

FIG. 2 shows an example of the rare cell capturing apparatus according to the present disclosure. The rare cell capturing apparatus shown in FIG. 2 includes an inlet 1 for supplying a specimen, a washing liquid, a staining liquid, and the like to a filter, an outlet 2, a channel 3 with which the inlet 1 and the outlet 2 are in communication, and an isolating portion that is arranged at a position corresponding to a portion of the channel 3. The isolating portion includes a filter 4 and a filter holder 5 for holding the filter 4. The filter holder 5 consists of a supporting portion (base) 6 on which the filter 4 can be mounted, an O-ring 7 for fixing the filter 4, and a cover 8. By mounting the filter 4 on the supporting portion (base) 6, putting the O-ring 7 and the cover 8 on the upper surface of the filter 4, and fixing the filter 4, the isolating portion (filtering device) provided with the filter with holes is formed. The channel 3, which connects the inlet 1 to the isolating portion, and the isolating portion to the outlet 2, is formed by connecting Safeed tubes (available from Terumo Corporation) to the two sides of the isolating portion. A connecting portion (not shown) and a three-way stopcock (not shown) capable of switching liquids may be arranged in the channel 3 connecting the inlet 1 to the isolating portion above the isolating portion. Moreover, a pressurizing means 9 (serving as a mechanism capable of performing feeding at a constant pressure or a mechanism capable of performing feeding at a constant flow amount) may be arranged in the channel 3 connecting the inlet 1 to the isolating portion to cause the pressure applied during filtration to be constant, or the feeding may be performed by performing sucking using a syringe pump such that the pressure applied during filtration is not greater than a certain pressure.

A kit or device for isolating a rare cell according to the present disclosure includes a filter of the present disclosure and a holding portion for holding the filter, and is configured to be attachable and detachable to and from a channel. In one or more embodiments, the kit or device for isolating a rare cell according to the present disclosure is used in the rare cell capturing apparatus and includes the filter of the present disclosure, the holding portion for holding the filter, and connecting portions. The connecting portions are capable of connecting the holding portion to the channel of the rare cell capturing apparatus, and are capable of passing the blood specimen from the rare cell capturing apparatus through the holding portion and the filter. The kit or device for isolating a rare cell is configured to be removably connected to and from the rare cell capturing apparatus. Therefore, in one or more embodiments of the rare cell capturing apparatus according to the present disclosure, the channel may include connecting portions that allow the kit or device for isolating a rare cell to be attachable and detachable. For example, the rare cell capturing apparatus shown in FIG. 2 may have two connecting portions: one is provided in a portion of the channel 3 between the pressurizing means 9 and the isolating portion, and the other is provided in a portion of the channel 3 between the isolating portion and the outlet 2. These connecting portions allow the isolating portion to be removably connected to and from the channel 3. The isolating portion that is configured to be attachable and detachable to and from the channel 3 is an example of the kit or device for isolating a rare cell according to the present disclosure. However, the kit or device for isolating a rare cell according to the present disclosure is not limited to this configuration.

EXAMPLES

Hereinafter, the present disclosure will be described more specifically by way of examples and comparative examples, but these are merely exemplary, and the present disclosure is not limited to these examples.

(Production of filter)

Various filters shown in Table 1 were produced. The holes of filters #3 to #14 had the slit shape shown in FIG. 1A, and the holes of filter #2 had the shape shown in FIG. 1B. The w ($\mu$m) in Table 1 is the major axis diameter of the hole, and z ($\mu$m) is the gap between the holes in the minor axis diameter direction (see FIG. 1A).

| (Table 1) | Filter | | | | | | | | | | |
|---|---|---|---|---|---|---|---|---|---|---|---|
| Filter # | Hole shape | Minor axis diameter (μm) × major axis diameter (μm) | Hole area (μm²) | w/z | Pitch between hole centers (minor axis diameter side × major axis diameter side) | Hole density (holes/mm²) | Opening rate (%) | Thickness (μm) | Material | Filtration area mm² | Total hole number |
| 1 | Circle | 6.5×6.5 | 33 | 0.9 | 14×19 | 3759 | 12 | 5 | Nickel (metal) | 20 | 73816 |
| 2 | Ellipse | 6.5×9.8 | 50 | 1.3 | 14×19 | 3759 | 19 | 5 | Nickel (metal) | 20 | 73816 |
| 3 | Slit | 5×88 | 440 | 9.8 | 14×100 | 714 | 31 | 5 | Nickel (metal) | 20 | 14025 |
| 4 | Slit | 6.5×88 | 572 | 7.3 | 18.5×100 | 541 | 31 | 5 | Nickel (metal) | 20 | 10613 |
| 5 | Slit | 8×88 | 704 | 6.1 | 22.5×100 | 444 | 31 | 5 | Nickel (metal) | 20 | 8727 |
| 6 | Slit | 6.5×40 | 260 | 5.3 | 16×52 | 1202 | 31 | 5 | Nickel (metal) | 20 | 23600 |
| 7 | Slit | 6.5×200 | 1300 | 15.4 | 19.5×212 | 242 | 31 | 5 | Nickel (metal) | 20 | 4750 |
| 8 | Slit | 6.5×500 | 3250 | 35.7 | 20.5×512 | 95 | 31 | 5 | Nickel (metal) | 20 | 1871 |
| 9 | Slit | 6.5×88 | 572 | 11.7 | 14×100 | 714 | 41 | 5 | Nickel (metal) | 20 | 14025 |
| 10 | Slit | 6.5×968 | 6292 | 129.1 | 14×1133 | 63 | 41 | 5 | Nickel (metal) | 20 | 1238 |
| 11 | Slit | 6.5×88 | 572 | 11.7 | 14×100 | 714 | 41 | 5 | Nickel (metal) | 79 | 56100 |
| 12 | Slit | 6.5×88 | 572 | 11.7 | 14×130 | 549 | 31 | 5 | Nickel (metal) | 20 | 10788 |
| 13 | Slit | 6.5×88 | 572 | 11.7 | 14×100 | 714 | 41 | 3 | Nickel (metal) | 79 | 56100 |

| (Table 1) | Filter | | | | | | | | | | |
|---|---|---|---|---|---|---|---|---|---|---|---|
| Filter # | Hole shape | Minor axis diameter ($\mu$m) $\times$ major axis diameter ($\mu$m) | Hole area ($\mu$m$^2$) | w/z | Pitch between hole centers (minor axis diameter side $\times$ major axis diameter side) | Hole density (holes/mm$^2$) | Opening rate (%) | Thickness ($\mu$m) | Material | Filtration area mm$^2$ | Total hole number |
| 14 | Slit | 6.5$\times$88 | 572 | 11.7 | 14$\times$100 | 714 | 41 | 15 | Nickel (metal) | 79 | 56100 |

The major axis diameter (w) and minor axis diameter of the filter were obtained by taking images of the holes at the center and the ends (deep, front, left, and right ends) of the filter with a microscope camera using a commercially available laser microscope VK-8510 (available from Keyence Corporation) equipped with a 10-fold to 100-fold objective lens, measuring the lengths of the long side and the short side of a rectangle circumscribed about each hole (50 or more holes) in the taken images by using software packaged with the microscope or commercially available image analysis software, and determining the averages. A metallographic microscope, an inverted microscope, and a microscope other than a laser microscope can also be used to perform the same measurements, and it is desirable to take images at a magnification that enables the measurement of a length of 1 $\mu$m or less.

The gap (z) between the holes was obtained by taking an image of the filter using a commercially available optical microscope equipped with a 10-fold to 100-fold objective lens, and measuring the shortest straight line that links the rectangles circumscribed about the holes adjacent to each other in the minor axis diameter direction in the taken image by using software packaged with the microscope (50 points). Alternatively, since the gaps between the holes are substantially uniform, the gap between the holes was obtained by measuring the minor axis diameters at the centers of the major axis diameters in the longitudinal direction (50 points), and determining the average.

The total number of holes was determined by taking an image of the filter using a commercially available optical microscope equipped with a 10-fold to 100-fold objective lens, and directly counting the number of holes included in the filter. Alternatively, the total number of holes was determined by multiplying the hole density per unit area by the filtration area.

The hole density was determined by measuring the lengths of pitches between the centers of the holes, and determining the number of holes per square millimeter from the average value of the lengths of pitches by calculation.

(Preparation of small cancer cell sample)

[SW620 sample]

According to a commonly used procedure, human colon cancer cells (SW620 cell line), which are adherent cells, were cultured in a petri dish, PBS(-) was added thereto after the culture medium was removed, and the cells were washed to remove the medium components and then subjected to a trypsin treatment (trypsin was available from Invitrogen) (37°C, 3 minutes). Culture medium supplemented with serum was added thereto, and the mixture was collected in a 15-ml centrifuge tube. The mixture was centrifuged using a centrifuge (CR20F: available from Hitachi), and the supernatant was removed. The cells were suspended in culture medium supplemented with serum again and collected. After being collected from the culture solution, the prepared cancer cell suspension was centrifuged at 1500 rpm at room temperature (24°C) for 3 minutes using a centrifuge (CR20F/available from Hitachi). The supernatant was removed, PBS(-) was added thereto, and the cells were suspended. These cells suspended in PBS(-) were fluorescently stained using a staining reagent CellTracker (available from Invitrogen). The CellTracker was dissolved in DMSO such that the concentration was 10 mM, and then added to the cell suspension such that the final concentration during the reaction was 0.5 to 0.25 $\mu$M. After the reaction, the cell suspension was centrifuged to remove the supernatant, and the cells were resuspended in PBS. This operation was repeated to remove the unreacted solution and wash the cells. Thereafter, the cells were suspended in culture medium or PBS(-) such that the cell suspension had a desired concentration (about $10^3$ cells/mL to $5 \times 10^4$ cells/mL).

Next, a blood specimen was prepared by placing a portion of the blood (containing 3000 to 10000 cells/$\mu$l of leukocytes) collected in a blood-collecting vessel (EDTA-2K) in a specimen container for blood filtration that was in communication with a filtration device, adding 10 $\mu$L of the stained cancer cell suspension to the blood in the specimen container for blood filtration, and then adding and mixing the remaining blood. The SW620 cells have a diameter of about 13 $\mu$m, and thus were used as a model of small cells.

(Preparation of sample of cancer cells having high deformability)

[SNU-1 sample]

Human stomach cancer cells (SNU-1 cell line), which are floating cells, were collected in a 15-ml centrifuge tube without performing a trypsin treatment. A blood specimen was prepared by performing the fluorescent staining and the addition to the blood in the same manner as in the preparation of the SW620 sample, except that the collected cancer cell suspension was used. Although the SNU-1 cells have a cell diameter of 16.8 $\mu$m, the SNU-1 cells more easily passed through the holes having a diameter of 6.5 $\mu$m than the SW620 cells.

[Colo320DM sample]

Since some human colorectal cancer cells (Colo320DM cell line) are floating cells and others are adherent cells, the floating cells were collected in a 15-ml centrifuge tube, and the adherent cells were collected in the same centrifuge tube after the trypsin treatment was performed in the same manner as with the SW620 cells. A blood specimen was prepared by performing the fluorescent staining and the addition to the blood in the same manner as in the preparation of the SW620 sample, except that the collected cancer cell suspension was used. Although the Colo320DM cells have a cell diameter of 14 $\mu$m, the Colo320DM more easily passed through the holes having a diameter of 6.5 $\mu$m than the SW620 cells.

In the following examples and comparative examples, the SNU-1 cells and the Colo320DM cells were used as models of cells that are likely to deform and pass through the holes (cells having high deformability).

(Comparative Example 1)

Filter #1 (major axis diameter: 6.5pm, minor axis diameter: 6.5pm, w/z: 0.9) was provided in the rare cell capturing apparatus shown in FIG. 2, and the filtration was performed under the following filtration conditions using the SW620 sample, the SNU-1 sample, and the Colo320DM sample to isolate and collect the cells. After the filtration, a buffer such as PBS(-) was fed at a $\Delta$P1 of 0.4 kPa to wash the filter, the connecting portion connected above the isolating portion was slowly removed, and then a glass slide was slowly put on the filter to produce an observation surface. This was mounted on a fluorescent microscope, and the number of fluorescently stained cancer cells remaining on the filter was counted. The same amount of the above-mentioned cancer cell suspension as that of the suspension added to the blood was placed in a well of a microplate, and the number of cancer cells was counted under the fluorescent microscope. The capture rate was calculated by dividing the number of cells remaining on the filter by the number of cells in the well of the microplate. Table 2 below shows the results.

<Filtration conditions>

Total treatment amount of blood specimen: 1 ml, 4 ml, 8 ml
Filtration pressure in the entire system ($\Delta$P1) during the specimen filtration: 1.3 kPa

| (Table 2) | Total treatment amount of blood | 1 ml | 4 ml | 8 ml |
|---|---|---|---|---|
| Small cells | SW620 | 100% | 93% | 101% |
| Cells having high deformability | SNU-1 | 56% | 46% | 29% |
| | Colo320DM | - | 73% | 37% |

Patent Document 5 (JP 2011-163830A) discloses that when a size-selective microcavity array having a diameter of 10 $\mu$m is used, the average collection rate for the SW620 cells is 38%. In contrast, it was found that with filter #1 with circular holes having a diameter of 6.5 $\mu$m, the SW620 cells could be captured at a high capture rate of more than 90%. On the other hand, the capture rates for the SNU-1 cells and the Colo320DM cells, which are cancer cells having a larger cell diameter than the SW620 cells but having high deformability, were lower than that of the SW620 cells. It was found that as the treatment amount of the blood increases, the capture rate for the cells having high deformability further decreases. Thus, it was found that the cancer cells having high deformability could not be captured sufficiently using the filter with circular holes having a diameter of 6.5 $\mu$m.

It is thought that the treatment amount of blood needs to be further reduced or the filtration area increased in order to maintain the high capture rates for both types of cells in filter #1. Alternatively, the hole density may be increased, but this is not desirable because the durability of the filter is adversely affected.

(Example 1)

In order to check the small cancer cell capture performance, each of filters #3, #4 and #5, which had the same opening rate and major axis diameter (w), and differed in the minor axis diameter, was provided in the rare cell capturing apparatus shown in FIG. 2, the filtration was performed under filtration condition 1 or 2 below using the SW620 sample to isolate and collect the cells, and the capture rate for the SW620 cells was determined. The isolation and collection of the cells, and the calculation of the capture rate were performed in the same manner as in Comparative Example 1.

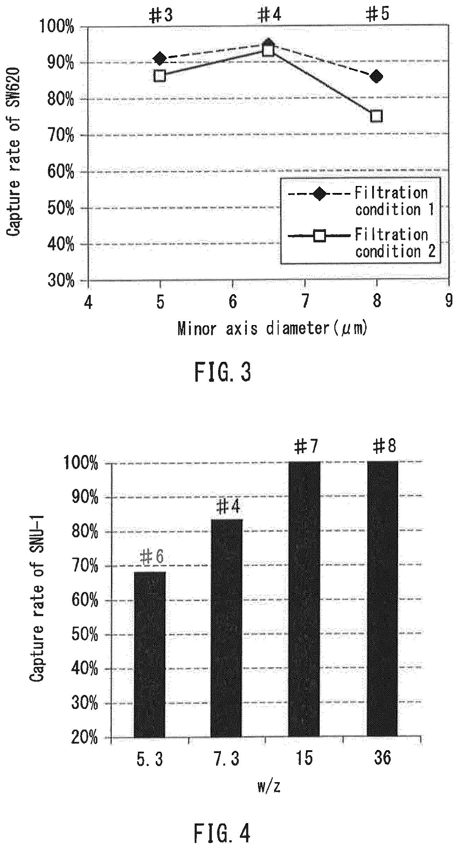

FIG. 3 shows the results. It should be noted that the difference between pressures on the upper side and the lower side of the filter ($\Delta P2$) was calculated based on the equation:

$$\Delta P_2 = Q/N_0 \times 12\eta L/[(1-0.63)(h/w)] \times (1/h^3 w)$$

where $\eta$ was 4.5 mPa as described above.

<Filter>

Filter #3 (minor axis diameter: 5.0 $\mu$m, major axis diameter: 88 $\mu$m, w/z: 9.8)
Filter #4 (minor axis diameter: 6.5 $\mu$m, major axis diameter: 88 $\mu$m, w/z: 7.3)
Filter #5 (minor axis diameter: 8.0 $\mu$m, major axis diameter: 88 $\mu$m, w/z: 6.1)

<Filtration conditions>

|  | Filtration condition 1 | Filtration condition 2 |
|---|---|---|
| Total treatment amount of blood specimen | 2 ml | 8 ml |
| Filtration pressure in entire system ($\Delta P1$) | 1.3 kPa | 0.4 kPa |
| Total average of flow amount | 1 ml/min to 1.3 ml/min | 0.09 ml/min to 0.13 ml/min |
| Treatment amount per square micrometer of hole area | 0.33 nl/$\mu$m$^2$ | 1.3 nl/$\mu$m$^2$ |
| Difference between pressures on upper side and lower side of filter (calculated value) ($\Delta P2$) | 12 Pa to 40 Pa | 1 Pa to 4 Pa |
| Average flow rate per hole | 160 mm/min to 220 mm/min | 14 mm/min to 22 mm/min |

FIG. 3 is a graph showing the relationship between the minor axis diameter of the hole and the capture rate for the small cells (SW620 cells). As shown in FIG. 3, when the treatment amount was 2 ml, the SW620 cells, which are cells having a relatively small diameter, could be captured at a capture rate of more than 80% with any of the filters. In particular, when filter #4 having a minor axis diameter of 6.5 $\mu$m was used, the capture rate was the highest. On the other hand, it was confirmed that in the case where the treatment amount was 8 ml, when filter #4 having a minor axis diameter of 6.5 $\mu$m was used, the capture rate was the highest, but when filter #5 having a minor axis diameter of 8 $\mu$m was used, the capture rate was less than 80%.

In the case where the SNU-1 cells, which are likely to deform, were used to perform the same measurements, when filter #3 having a minor axis diameter of 5 $\mu$m was used, the capture rate was the lowest (data not shown).

(Example 2)

The capture of cells and the calculation of the capture rate were performed in the same manner as in Example 1, except that each of filters #6, #4, #7 and #8, which had the same minor axis diameter and opening rate (31%), was used and the filtration was performed under the following filtration conditions. The SNU-1 sample was used as a sample. FIG. 4 shows the results.

<Filter>

Filter #6 (minor axis diameter: 6.5 $\mu$m, major axis diameter: 40 $\mu$m, w/z: 5.3)

Filter #4 (minor axis diameter: 6.5 $\mu$m, major axis diameter: 88 $\mu$m, w/z: 7.3)
Filter #7 (minor axis diameter: 6.5 $\mu$m, major axis diameter: 200 $\mu$m, w/z: 15.4)
Filter #8 (minor axis diameter: 6.5 $\mu$m, major axis diameter: 500 $\mu$m, w/z: 35.7)

<Filtration conditions>

| | |
|---|---|
| Total treatment amount of blood specimen | 4 ml |
| Filtration pressure in entire system ($\triangle$P1) | 0.4 kPa |
| Total average of flow amount | 0.11 ml/min to 0.16 ml/min |
| Treatment amount per square micrometer of hole area | 0.7 nl/$\mu$m$^2$ |
| Difference between pressures on upper side and lower side of filter (calculated value) ($\triangle$P2) | 2 Pa to 3 Pa |
| Average flow rate per hole | 18 mm/min to 26 mm/min |

FIG. 4 is a graph showing the relationship between the w/z and the capture rate. As shown in FIG. 4, the SNU-1 cells could be collected at a high capture rate of more than 80% with any of the filters having a w/z of 7.0 or more. It was confirmed that the SNU-1 cells could be collected at a higher capture rate as the w/z value of the filter increased even in the case where the opening rates were the same. Moreover, since the SNU-1 cells could be collected at a low filtration pressure ($\triangle$P1), which is the pressure in the entire system during filtering, of 0.4 kPa (calculated value of the difference between the pressures on the upper side and the lower side of the filter ($\triangle$P2): 2 Pa to 3 Pa), it is thought that damage to the collected rare cells can be reduced.

(Example 3)

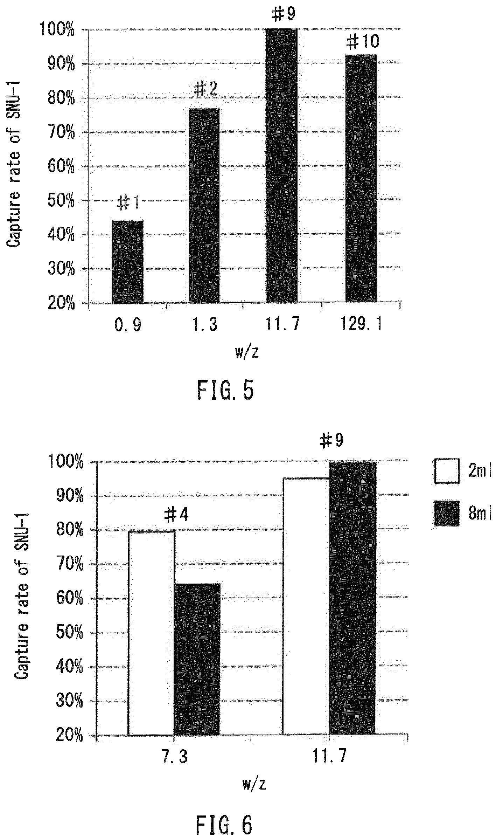

The capture of cells and the calculation of the capture rate were performed in the same manner as in Example 2, except that each of filters #9 and #10, which had the same minor axis diameter (6.5 pm) and z (length of the gap between the holes in the minor axis diameter direction) (7.5 pm), was used and the filtration was performed under filtration condition 3 or 4 below. FIG. 5 shows the results. It should be noted that as reference examples, the capture of cells and the calculation of the capture rate were performed in the same manner, except that each of filter #1 (the gap (z) between the holes in the minor axis diameter direction was 7.5 pm) having circular holes and filter #2 (the gap (z) between the holes in the minor axis diameter direction was 7.5 $\mu$m) having elliptical holes, was used and the filtration was performed under reference filtration condition 1 or 2 below.

<Filter>

Filter #1 (minor axis diameter: 6.5 $\mu$m, major axis diameter: 6.5 $\mu$m, w/z: 0.9)
Filter #2 (minor axis diameter: 6.5 $\mu$m, major axis diameter: 9.8 $\mu$m, w/z: 1.3)
Filter #9 (minor axis diameter: 6.5 $\mu$m, major axis diameter: 88 $\mu$m, w/z: 11.7)
Filter #10 (minor axis diameter: 6.5 $\mu$m, major axis diameter: 968 $\mu$m, w/z: 129.1)

<Filtration conditions>

| | Ex. | | Ref Ex. | |
|---|---|---|---|---|
| | Filtration condition 3 | Filtration condition 4 | Reference filtration condition 1 | Reference filtration condition 2 |
| Type of filter | #9 | #10 | #1 | #2 |
| w/z | 11.7 | 129.1 | 0.9 | 1.3 |

(continued)

| | Ex. | | Ref Ex. | |
|---|---|---|---|---|
| | Filtration condition 3 | Filtration condition 4 | Reference filtration condition 1 | Reference filtration condition 2 |
| Type of filter | #9 | #10 | #1 | #2 |
| Total treatment amount of blood specimen | 8 ml | 8 ml | 8 ml | 8 ml |
| Filtration pressure in entire system ($\Delta P1$) | 0.4 kPa | 0.4 kPa | 0.4 kPa | 0.4 kPa |
| Total average of flow amount | 0.07 ml/min to 0.13 ml/min | 0.07 ml/min to 0.13 ml/min | 0.07 ml/min to 0.13 ml/min | 0.07 ml/min to 0.13 ml/min |
| Treatment amount per square micrometer of hole area | 1 nl/$\mu$m$^2$ | 1 nl/$\mu$m$^2$ | 3.3 nl/$\mu$m$^2$ | 2.2 nl/$\mu$m$^2$ |
| Difference between pressures on upper side and lower side of filter (calculated value) ($\Delta P2$) | 1 Pa to 2 Pa | 1 Pa to 2 Pa | 8 Pa to 15 Pa | 4 Pa to 8 Pa |
| Average flow rate per hole | 11 mm/min to 17 mm/min | 11 mm/min to 17 mm/min | 27 mm/min to 54 mm/min | 24 mm/min to 36 mm/min |

FIG. 5 is a graph showing the relationship between the w/z and the capture rate. As shown in FIG. 5, the SNU-1 cells could be collected at a high capture rate of more than 90% with filters #9 and #10 (w/z is 7 or more). In particular, with filter #9 (major axis diameter: 88 $\mu$m, w/z: 11.7), the occurrence of bending and distortion was suppressed compared with filter #10, and the SNU-1 cells could be captured at a capture rate close to 100%.

(Example 4)

The capture of cells and the calculation of the capture rate were performed in the same manner as in Example 2, except that each of filters #4 and #9, which had the same minor axis diameter and major axis diameter (w), was used and the filtration was performed under any of filtration conditions 5 to 8 below. FIG. 6 shows the results.

<Filter>

Filter #4 (minor axis diameter: 6.5 $\mu$m, major axis diameter: 88 $\mu$m, w/z: 7.3)
Filter #9 (minor axis diameter: 6.5 $\mu$m, major axis diameter: 88 $\mu$m, w/z: 11.7)

<Filtration conditions>

| | Filtration condition 5 | Filtration condition 6 | Filtration condition 7 | Filtration condition 8 |
|---|---|---|---|---|
| Type of filter | #4 | | #9 | |
| w/z | 7.3 | 7.3 | 11.7 | 11.7 |
| Total treatment amount of blood specimen | 2 ml | 8 ml | 2 ml | 8 ml |
| Filtration pressure in entire system ($\Delta P1$) | 0.4 kPa | 0.4 kPa | 0.4 kPa | 0.4 kPa |

(continued)

| | Filtration condition 5 | Filtration condition 6 | Filtration condition 7 | Filtration condition 8 |
|---|---|---|---|---|
| Type of filter | #4 | | #9 | |
| Total average of flow amount | 0.15 ml/min to 0.25 ml/min | 0.07 ml/min to 0.13 ml/min | 0.15 ml/min to 0.25 ml/min | 0.07 ml/min to 0.13 ml/min |
| Treatment amount per square micrometer of hole area | $0.33 \text{ nl}/\mu m^2$ | $1.3 \text{ nl}/\mu m^2$ | $0.25 \text{ nl}/\mu m^2$ | $1 \text{ nl}/\mu m^2$ |
| Difference between pressures on upper side and lower side of filter (calculated value) ($\Delta P2$) | 2 Pa to 5 Pa | 1 Pa to 3 Pa | 2 Pa to 4 Pa | 1 Pa to 3 Pa |
| Average flow rate per hole | 27 mm/min to 41 mm/min | 11 mm/min to 22 mm/min | 21 mm/min to 31 mm/min | 11 mm/min to 17 mm/min |

FIG. 6 is a graph showing the relationship between the w/z and the capture rate. In FIG. 6, the white bars show the results of the cases where the total treatment amount of the blood specimen was 2 ml (filtration conditions 5 and 7), and the black bars show the results of the cases where the total treatment amount of the blood specimen was 8 ml (filtration conditions 6 and 8). As shown in FIG. 6, with filter #4 having a w/z of 7.3, when the total treatment amount of the blood specimen was 8 ml, the capture rate decreased, but when the total treatment amount of the blood specimen was 2 ml, the capture rate was high. Surprisingly, with filter #9 having a w/z of 11.7, the SNU-1 cells could be captured at a high capture rate close to 100% regardless of the total treatment amount of the blood specimen.

From the results of Examples 1 to 4 in which one or two of the opening rate, the minor axis diameter, the major axis diameter and the z were fixed and the w/z was varied, it was confirmed that setting the w/z to 7 or more made it possible to improve the capture rate for the cell having high deformability, and furthermore, to improve the capture rate even when the total treatment amount of blood was increased.

(Example 5)

The capture of cells and the calculation of the capture rate were performed in the same manner as in Example 2, except that filter #9 (minor axis diameter: 6.5 $\mu$m, major axis diameter: 88 $\mu$m, w/z: 11.7) was used and the total treatment amount of the blood specimen was varied. The pressure during the filtration was as follows. Table 7 shows the results.

<Filtration condition>

Filtration pressure in entire system ($\Delta P1$): 0.4 kPa

(Table 7)

| Addition amount (total treatment amount of blood specimen) | 1 ml | 4 ml | 8 ml | 10 ml | 12 ml | 25 ml |
|---|---|---|---|---|---|---|
| Blood amount per hole (nl) | 71 | 285 | 570 | 713 | 856 | 1783 |
| Blood amount per hole area ($\text{nl}/\mu m^2$) | 0.1 | 0.5 | 1.0 | 1.2 | 1.5 | 3.1 |
| Capture rate | - | 92% | 100% | 90% | 92% | 72% |

As shown in Table 7, the SNU-1 cells could be captured at a capture rate of more than 70% in all cases. Moreover, it was confirmed that setting the treatment amount per hole area to be in a range of more than 0.1 $\text{nl}/\mu m^2$ to 3.1 $\text{nl}/\mu m^2$ or less made it possible to collect the SNU-1 at a higher capture rate.

(Example 6)

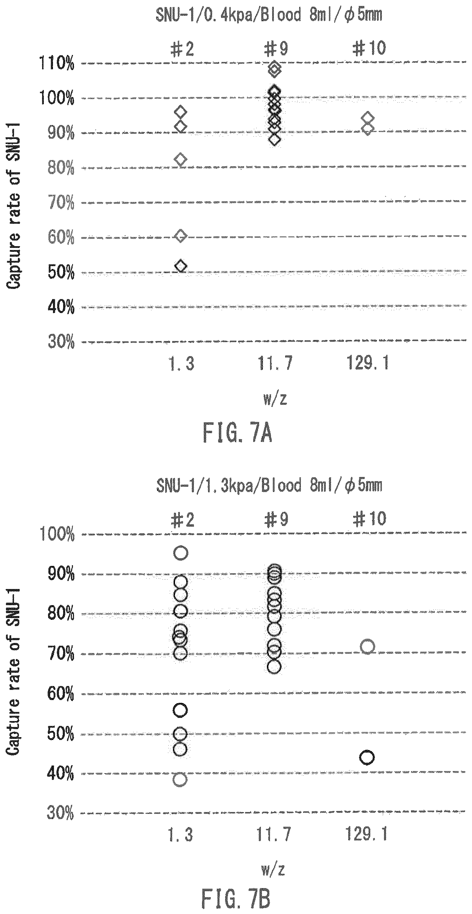

The capture of cells and the calculation of the capture rate were performed in the same manner as in Example 2, except that filter #9 or #10 was used, the total treatment amount of the blood specimen was 8 ml, and the filtration was performed under any of filtration conditions 9 to 12 below. FIG. 7 shows the results. It should be noted that as reference examples, the capture of cells and the calculation of the capture rate were performed in the same manner, except that filter #2 having elliptical holes was used and the filtration was performed under reference filtration condition 3 or 4 below.

<Filter>

Filter #2 (minor axis diameter: 6.5 $\mu$m, major axis diameter: 9.8 $\mu$m, w/z: 1.5)
Filter #9 (minor axis diameter: 6.5 $\mu$m, major axis diameter: 88 $\mu$m, w/z: 11.7)
Filter #10 (minor axis diameter: 6.5$\mu$m, major axis diameter: 968 $\mu$m, w/z: 129.1)

<Filtration conditions>

| | Ref. Ex. | Ex. | | Ref. Ex. | Ex. | |
|---|---|---|---|---|---|---|
| | Reference filtration condition 3 | Filtration condition 9 | Filtration condition 10 | Reference filtration condition 4 | Filtration condition 11 | Filtration condition 12 |
| Type of filter | #2 | #9 | #10 | #2 | #9 | #10 |
| w/z | 1.3 | 11.7 | 129.1 | 1.3 | 11.7 | 129.1 |
| Total treatment amount of blood specimen | 8 ml | 8 ml | 8 ml | 8 ml | 8 ml | 8 ml |
| Filtration pressure in entire system ($\Delta$P1) | 0.4 kPa | 0.4 kPa | 0.4 kPa | 1.3 kPa | 1.3 kPa | 1.3 kPa |
| Total average of flow amount | 0.07 ml/min to 0.13 ml/min | 0.07 ml/min to 0.13 ml/min | 0.07 ml/min to 0.13 ml/min | 0.3 ml/min to 0.6 ml/min | 0.3 ml/min to 0.6 ml/min | 0.3 ml/min to 0.6 ml/min |
| Treatment amount per square micrometer of hole area | 2.2 nl/$\mu$m$^2$ | 1 nl/$\mu$m$^2$ | 1 nl/$\mu$m$^2$ | 2.2 nl/$\mu$m$^2$ | 1 nl/$\mu$m$^2$ | 1 nl/$\mu$m$^2$ |
| Difference between pressures on upper side and lower side of filter (calculated value) ($\Delta$P2) | 4 Pa to 8 Pa | 1 Pa to 2 Pa | 1 Pa to 2 Pa | 40 Pa to 60 Pa | 4 Pa to 9 Pa | 4 Pa to 9 Pa |
| Average flow rate per hole | 18 mm/min to 36 mm/min | 11 mm/min to 17 mm/min | 11 mm/min to 17 mm/min | 90 mm/min to 170 mm/min | 40 mm/min to 80 mm/min | 40 mm/min to 80 mm/min |

FIG. 7 is a graph showing the variations in the capture rates of the filters. FIG. 7A shows the results of the case where the filtration pressure in the entire system ($\Delta$P1) was 0.4 kPa (filtration conditions 9 and 10, and reference filtration condition 3), and FIG. 7B shows the results of the case where the filtration pressure in the entire system ($\Delta$P1) was 1.3 kPa (filtration conditions 11 and 12, and reference filtration condition 4). It was confirmed that as shown in FIGS. 7A and 7B, with filters #9 and #10, the variations were smaller and the difference between the specimens was smaller compared with filter #2 under both pressure conditions. It was confirmed that particularly with filter #9, the capture rate was high, and the variations were very small under both pressure conditions.

The capture of cells and the calculation of the capture rate were performed in the same manner as in Examples 2 to 6, except that the SW620 sample was used instead of the SNU-1 sample. It was confirmed that the SW620 cells could be captured at a high capture rate of more than 90% or close to 90%, with any of the filters or under the filtration conditions (data not shown).

(Example 7)

The capture of cells and the calculation of the capture rate were performed using the SNU-1 cells, the SW620 cells, the Colo320DM cells, human lung cancer cells (NCI-H1703 cell line), human lung cancer cells (A549 cell line), human lung cancer cells (NCI-H1975 cell line), human lung cancer cells (NCI-H69 cell line), and human lung cancer cells (NCI-H1603 cell line) as cancer cell samples. Blood specimen samples were prepared in the same manner as in the above-mentioned preparation of the small cancer cell sample. Moreover, when cells forming a large number of aggregates (e.g., NCI-H69 cells) are used, the number of cells counted may vary, and therefore, a filtrate that was obtained by dispersing the cells using a dispersing agent FACSmax (available from Genlantis) according to the protocol, and then removing remaining aggregates that were not dispersed using a nylon mesh filter (available from Millipore) with 20-$\mu$m holes having a filtration area diameter of 13 mm was used as a cell suspension. As the filters, filter #9 and #11 (w/z=11.7, major axis diameter=88 $\mu$m, minor axis diameter=6.5 pm), which had different filtration areas but had the same minor axis diameter, major axis diameter, and w/z, were used. The filtration conditions were as follows. Tables 10 and 11 below show the results.

<Filtration conditions>

| Type of filter | #9 | #11 |
|---|---|---|
| w/z | 11.7 | 11.7 |
| Total treatment amount of blood specimen | 8 ml | 8 ml |
| Filtration pressure in entire system ($\Delta$P1) | 0.4 kPa | Constant flow amount feeding (syringe pump feeding) |
| Total average of flow amount | 0.07 ml/min to 0.13 ml/min | 0.25 ml/min |
| Treatment amount per square micrometer of hole area | 1 nl/$\mu$m$^2$ | 0.25 nl/$\mu$m$^2$ |
| Difference between pressures on upper side and lower side of filter (calculated value) ($\Delta$P2) | 1 Pa to 2 Pa | 1 Pa |
| Average flow rate per hole | 11 mm/min to 17 mm/min | 8 mm/min |

| (Table 10) Filter #9 | Lung cancer | | | | Colorectal cancer | | Stomach cancer |
|---|---|---|---|---|---|---|---|
| | NCI-H1703 | A549 | NCI-H1975 | NCI-H69 | Colo320DM | SW620 | SNU-1 |
| Cell size | 16.2 $\mu$m | 16.2 $\mu$m | 18.1 $\mu$m | 12.4 $\mu$m | 14 $\mu$m | 13 $\mu$m | 16.8 $\mu$m |
| Capture rate | 98.8% | 96.5% | 96.4% | 87.7% | 97.5% | 95% | 99.6% |
| SD | - | - | - | 8% | 12% | 6% | 7% |

| (Table 11) Filter#11 | Lung cancer | | | | | Colorectal cancer | Stomach cancer |
|---|---|---|---|---|---|---|---|
| | NCI-H1703 | A549 | NCI-H1975 | NCI-H69 | NCI-H1650 | SW620 | SNU-1 |
| Cell size | 16.2 $\mu$m | 16.2 $\mu$m | 18.1 $\mu$m | 12.4 $\mu$m | 18.6 $\mu$m | 13 $\mu$m | 16.8 $\mu$m |
| Capture rate | 98% | 103% | 94% | 95% | 91% | 95% | 94% |
| SD | 10% | 3% | 3% | 3% | 5% | 6% | 7% |

Table 10 shows the capture rates of filter #9 (filtration area: 20 mm$^2$), and Table 11 shows the capture rates of filter #11 (filtration area: 79 mm$^2$). As shown in Tables 10 and 11 above, various cells could be efficiently captured regardless of the type of cells such as floating cells and adherent cells. Moreover, various cells could be efficiently captured regardless of the filtration area.

(Example 8)

Cancer cells were captured using a high-Hct specimen (i.e., a specimen having a high Hct value).

First, a high-Hct sample was prepared. The collected blood was divided into two 15-ml centrifuge tubes, and was centrifuged using a centrifuge at 1500×g for about 10 minutes. After centrifugation, the separated layer of the blood plasma was transferred to another 15-ml centrifuge tube. The erythrocyte layer in one of the two centrifuge tubes containing only a hemocyte layer was transferred to the other centrifuge tube, and then the separated blood plasma was added thereto such that the Hct value was 60 or more to prepare a high-Hct blood specimen. Next, a high-Hct sample was prepared in the same manner as the above-mentioned SNU-1 sample, except that the prepared high-Hct blood specimen was used instead of blood. It should be noted that the Hct value of the prepared high-Hct blood sample was determined using a hemocyte counter SB-1440 (available from Arkray Inc.).

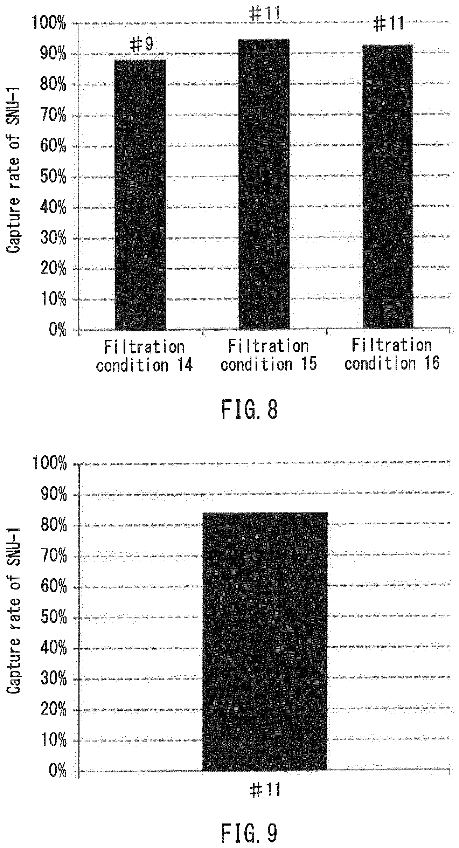

The capture of cells and the calculation of the capture rate were performed in the same manner as in Example 1, except that each of the prepared high-Hct samples (three types) and filters # 9 and #11 were used and the filtration was performed under any of filtration conditions 13 to 15 below. FIG. 8 shows the results.

| | Filtration condition 13 | Filtration condition 14 | Filtration condition 15 |
|---|---|---|---|
| Hct | 62% | 65% | 63% |
| Type of filter | #9 | #11 | #11 |
| w/z | 11.7 | 11.7 | 11.7 |
| Total treatment amount of blood specimen | 8 ml | 8 ml | 8 ml |
| Filtration pressure in entire system (ΔP1) | 0.4 kPa | 0.75 kPa | Constant flow amount feeding using syringe pump |
| Total average of flow amount | 0.05 ml/min | 0.13 ml/min | 0.25 ml/min |
| Treatment amount per square micrometer of hole area | 1 nl/μm$^2$ | 0.25 nl/μm$^2$ | 0.25 nl/μm$^2$ |
| Difference between pressures on upper side and lower side of filter (calculated value) (ΔP2) | 0.5 Pa | 0.6 Pa | 1 Pa |
| Average flow rate per hole | 6 mm/min | 4 mm/min | 8 mm/min |

As shown in FIG. 8, even when the high-Hct samples having an Hct of more than 60% were used, the SNU-1 cells could be captured at a high capture rate of more than 90% or close to 90%.

(Example 9)

Cancer cells were captured using a high-leukocyte specimen (i.e., a specimen including a large number of leukocytes).

First, a high-leukocyte sample was prepared. Leukocytes that had been isolated and collected using HetaSep (available from STEMCELL Technologies) were mixed with blood to prepare a high-leukocyte blood specimen. Next, a high-leukocyte sample was prepared in the same manner as the above-mentioned SNU-1 sample, except that the prepared high-leukocyte blood specimen was used instead of blood. It should be noted that the number of leukocytes in the prepared high-leukocyte sample was determined using a hemocyte counter SB-1440 (available from Arkray Inc.).

The capture of cells and the calculation of the capture rate were performed in the same manner as in Example 1, except that the prepared high-leukocyte sample and filter #11 were used and the filtration was performed under the following filtration conditions. FIG. 9 shows the results.

<Filtration conditions>

| Leukocyte number | $174\times10^2$ cells/$\mu$L |
|---|---|
| Type of filter | #11 |
| w/z | 11.7 |
| Total treatment amount of blood specimen | 8 ml |
| Filtration pressure in entire system ($\triangle$P1) | 0.75 kPa |
| Total average of flow amount | 0.04 ml/min |
| Treatment amount per square micrometer of hole area | 0.25 nl/$\mu$m$^2$ |
| Difference between pressures on upper side and lower side of filter (calculated value) ($\triangle$P2) | 1.4 Pa |
| Average flow rate per hole | 12 mm/min |

As shown in FIG. 9, even when the high-leukocyte sample in which the number of leukocytes was more than 10,000 cells /$\mu$L was used, the SNU-1 cells could be captured at a high capture rate of more than 80%.

(Comparative Example 2)

A filter having the same hole arrangement (minor diameter of 8 $\mu$m $\times$ major diameter of 100 $\mu$m, w/z=6.7, filtration area: 20 mm$^2$) as that of the filter described in Non-Patent Document 6 was used to evaluate the capture rates for the SW620 sample, the SNU-1 sample, and the NCI-H69 sample. The filtration was performed under filtration conditions such that 8 ml of each blood specimen sample was fed at 0.4 kPa, and the capture of cells and the calculation of the capture rate were performed in the same manner as in Example 1. Table 14 below shows the obtained capture rates. Moreover, the capturing experiment was performed using filter #9 under the same filtration conditions. Table 14 below also shows the results.

| (Table 14) | Colorectal cancer | Stomach cancer | Lung cancer |
|---|---|---|---|
| | SW620 | SNU-1 | NCI-H69 |
| Filter of Comp. Ex. 2 | 75% | 65% | 46% |
| Filter #9 (Ex.) | 95% | 98% | 93% |

As shown in Table 14 above, in the case where filter #9 (minor axis diameter: 6.5 $\mu$m, major axis diameter: 88 $\mu$m, w/z: 11.7) was used, the capture rate was more than 90%, and all of the capture rates for the colorectal cancer cells, the stomach cancer cells, and the lung cancer cells were higher compared with the filter disclosed in Non-Patent Document 6. Therefore, it can be said that use of filters such as filter #9 according to the present disclosure makes it possible to achieve the improvement of the capture rates for a small cancer cell and a cell that is likely to deform compared with the filter disclosed in Non-Patent Document 6.

(Example 10)

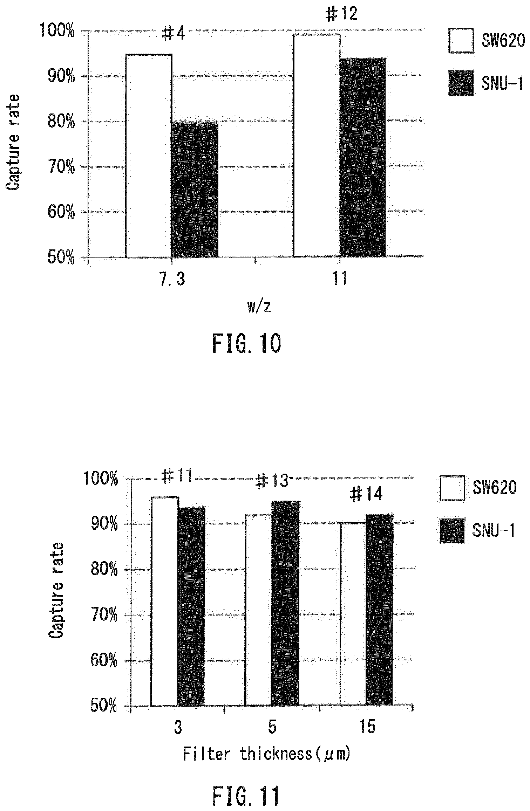

The capture of cells and the calculation of the capture rate were performed in the same manner as in Example 1, except that each of filters #4 and #12, which had the same opening rate, minor axis diameter, and major axis diameter (w), was used, the SW620 sample and the SNU-1 sample were used, and the filtration was performed under filtration condition 16 below. FIG. 10 shows the results.

<Filter>

Filter #4 (minor axis diameter: 6.5 $\mu$m, major axis diameter: 88 $\mu$m, w/z: 7.3)

Filter #12 (minor axis diameter: 6.5 $\mu$m, major axis diameter: 88 $\mu$m, w/z: 11.7)

<Filtration conditions>

| | Filtration condition 16 |
|---|---|
| Total treatment amount of blood specimen | 4ml |
| Filtration pressure in entire system ($\triangle$P1) | 0.4 kPa |
| Total average of flow amount | 0.11 ml/min to 0.16 ml/min |
| Treatment amount per square micrometer of hole area | 0.7 nl/$\mu$m$^2$ |
| Difference between pressures on upper side and lower side of filter (calculated value) ($\triangle$P2) | 2 Pa to 3 Pa |
| Average flow rate per hole | 18 mm/min to 26 mm/min |

FIG. 10 is a graph showing the relationship between the w/z and the capture rate. In FIG. 10, the white bars show the results of the cases where the SW620 sample was used, and the black bars show the results of the cases where the SNU-1 sample was used. As shown in FIG. 10, when the w/z was 7 or more, the SW620 cells and the SNU-1 cells could be captured at a capture rate of 80% or more, and in particular, when the w/z was 11 or more, the SW620 cells and the SNU-1 cells could be captured at a high capture rate of 90% or more.

(Example 11)

The capture of cells and the calculation of the capture rate were performed in the same manner as in Example 1, except that each of filters #11, #13, and #14, which differed in thickness, was used, the SW620 sample and the SNU-1 sample were used, and the filtration was performed under filtration condition 17 below. FIG. 11 shows the results.

<Filter>

Filter #11 (major axis diameter: 88 $\mu$m, w/z: 11.7, thickness: 5 $\mu$m)
Filter #13 (major axis diameter: 88 $\mu$m, w/z: 11.7, thickness: 3 $\mu$m)
Filter #14 (major axis diameter: 88 $\mu$m, w/z: 11.7, thickness: 15 $\mu$m)

<Filtration conditions>

| | Filtration condition 17 |
|---|---|
| w/z | 11.7 |
| Total treatment amount of blood specimen | 8 ml |
| Filtration pressure in entire system ($\triangle$P1) | 0.75 kPa |
| Total average of flow amount | 0.25 ml/min to 0.5 ml/min |
| Treatment amount per square micrometer of hole area | 0.25 nl/$\mu$m$^2$ |
| Difference between pressures on upper side and lower side of filter (calculated value) ($\triangle$P2) | 1 Pa to 2 Pa |
| Average flow rate per hole | 8 mm/min to 16 mm/min |

FIG. 11 is a graph showing the relationship between the filter thickness and the capture rate. In FIG. 11, the white bars show the results of the cases where the SW620 sample was used, and the black bars show the results of the cases where the SNU-1 sample was used. As shown in FIG. 11, even when any of the filters, which differed in thickness, was used, the SW620 cells and the SNU-1 cells could be captured at a high capture rate of more than 90%.

(Example 12)

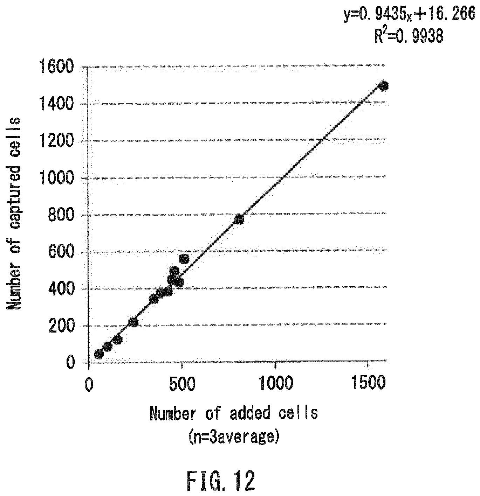

The capture of cells and the calculation of the capture rate were performed in the same manner as in Example 1, except that filter #9 was used, a plurality of SNU-1 samples that differed in the number of cells contained therein were used, and the filtration was performed under filtration condition 18 below. FIG. 12 shows the results.

<Filtration conditions>

| | Filtration condition 18 |
| --- | --- |
| w/z | 11.7 |
| Total treatment amount of blood specimen | 8 ml |
| Filtration pressure in entire system ($\triangle$P1) | 0.4 kPa |
| Total average of flow amount | 0.07 ml/min to 0.13 ml/min |
| Treatment amount per square micrometer of hole area | 1 nl/$\mu$m$^2$ |
| Difference between pressures on upper side and lower side of filter (calculated value) ($\triangle$P2) | 1 Pa to 2 Pa |
| Average flow rate per hole | 11 mm/min to 17 mm/min |

FIG. 12 is a graph showing the relationship between the number of cells contained in the sample and the number of captured cells. As shown in FIG. 12, the SNU-1 cells could be captured at a high capture rate regardless of the number of cells contained in the sample.

**Claims**

1. A method for isolating or detecting a rare cell, comprising filtering a blood specimen using a filter to isolate or detect a rare cell in the blood specimen,
   wherein the filter includes holes having a minor axis diameter of 5 $\mu$m to 8 $\mu$m and a major axis diameter of 40 $\mu$m or more at a hole density of 40 holes/mm$^2$ to 2000 holes/mm$^2$
   **characterized in that**,
   the ratio (w/z) between the major axis diameter w ($\mu$m) and a gap z ($\mu$m) between the holes in the minor axis diameter direction being 7.0 to 130.

2. The method according to claim 1, wherein the blood specimen is filtered such that the treatment capacity per hole area of the filter is 0.1 nl/$\mu$m$^2$ to 3 nl/$\mu$m$^2$.

3. The method according to claim 1 or 2, wherein the filter has an opening rate of 10 % to 60%.

4. The method according to any of claims 1 to 3, wherein the filtration pressure during filtering, which is the pressure difference in the entire system from the entrance of the system to the exit of the system, is 0.1 kPa to 2.6 kPa.

5. The method according to any of claims 1 to 4, wherein the difference between pressures on the upper surface and the lower surface of the filter during the filtering is 100 Pa or less.

6. The method according to any of claims 1 to 5, comprising feeding the blood specimen to the filter at an average flow rate of 1 mm/min to 600 mm/min per hole of the filter.

7. The method according to any of claims 1 to 6, wherein the rare cell is a cell selected from the group consisting of a cancer cell, a circulating tumor cell, a vascular endothelial cell, a vascular endothelial precursor cell, a cancer stem cell, an epithelial cell, a hematopoietic stem cell, a mesenchymal stem cell, a fetal cell, and combinations thereof.

8. A method for analyzing a rare cell in a blood specimen, comprising analyzing a rare cell with a method including observing the kinetics of the cell or measuring activity of the cell, or with a gene analysis of the cell after the rare cell is isolated or detected with the method according to any of claims 1 to 7.

9. A filter suitable for use in the method according to any of claims 1 to 7, the filter including holes having a minor axis diameter of 5 $\mu$m to 8 $\mu$m and a major axis diameter of 40 $\mu$m or more at a hole density of 40 holes/mm$^2$ to 2000 holes/mm$^2$,
**characterized in that**,
the ratio (w/z) between the major axis diameter w ($\mu$m) and a gap z ($\mu$m) between the holes in the minor axis diameter direction being 7.0 to 130.

10. A rare cell capturing apparatus for capturing a rare cell contained in a specimen, the apparatus comprising:

an inlet;
an outlet;
a channel with which the inlet and the outlet are in communication; and
an isolating portion,
wherein the isolating portion is arranged at a position corresponding to a portion of the channel, and includes the filter according to claim 9 and a holding portion for holding the filter.

11. Use of a filter for isolating, detecting or analyzing a rare cell in a blood specimen, wherein said filter is as defined in claim 9.

12. A kit or a device for isolating or detecting a rare cell detachably connected to a flow channel comprising a filter according to claim 9 and a filter-holding portion.


**Patentansprüche**

1. Verfahren zur Isolierung oder Detektion einer seltenen Zelle, umfassend ein Filtern einer Blutprobe unter Verwendung eines Filters, um eine seltene Zelle in der Blutprobe zu isolieren oder zu detektieren,
wobei das Filter Löcher beinhaltet, die einen Nebenachsendurchmesser von 5 $\mu$m bis 8 $\mu$m und einen Hauptachsendurchmesser von 40 $\mu$m oder mehr bei einer Lochdichte von 40 Löcher/mm$^2$ bis 2000 Löcher/mm$^2$ aufweisen,
**dadurch gekennzeichnet, dass**
das Verhältnis (w/z) zwischen dem Hauptachsendurchmesser w ($\mu$m) und einem Spalt z ($\mu$m) zwischen den Löchern in der Nebenachsendurchmesserrichtung 7,0 bis 130 ist.

2. Verfahren nach Anspruch 1, wobei die Blutprobe so gefiltert wird, dass die Behandlungskapazität pro Lochbereich des Filters 0,1 nl/$\mu$m$^2$ bis 3 nl/$\mu$m$^2$ ist.

3. Verfahren nach Anspruch 1 oder 2, wobei das Filter eine Öffnungsrate von 10 % bis 60 % aufweist.

4. Verfahren nach einem der Ansprüche 1 bis 3, wobei der Filterungsdruck während der Filterung, der der Druckunterschied in dem gesamten System vom Eingang des Systems bis zum Ausgang des Systems ist, 0,1 kPa bis 2,6 kPa ist.

5. Verfahren nach einem der Ansprüche 1 bis 4, wobei der Unterschied zwischen Drücken an der oberen Oberfläche und der unteren Oberfläche des Filters während der Filterung 100 Pa oder weniger ist.

6. Verfahren nach einem der Ansprüche 1 bis 5, umfassend ein Zuführen der Blutprobe zu dem Filter bei einer Durchschnittsflussrate von 1 mm/min bis 600 mm/min pro Loch des Filters.

7. Verfahren nach einem der Ansprüche 1 bis 6, wobei die seltene Zelle eine Zelle ist, die aus der Gruppe ausgewählt ist, bestehend aus einer Krebszelle, einer zirkulierenden Tumorzelle, einer vaskulären Endothelzelle, einer vaskulären Endothelvorläuferzelle, einer Krebsstammzelle, einer Epithelzelle, einer blutbildenden Stammzelle, einer mesenchymalen Stammzelle, einer Embryonalzelle und Kombinationen davon.

8. Verfahren zum Analysieren einer seltenen Zelle in einer Blutprobe, umfassend ein Analysieren einer seltenen Zelle mit einem Verfahren, das Beobachten der Kinetik der Zelle oder Messen von Aktivität der Zelle beinhaltet, oder mit einer Genanalyse der Zelle, nachdem die seltene Zelle mit dem Verfahren nach einem der Ansprüche 1 bis 7 isoliert oder detektiert wird.

9. Filter, das zur Verwendung in dem Verfahren nach einem der Ansprüche 1 bis 7 geeignet ist, wobei das Filter Löcher beinhaltet, die einen Nebenachsendurchmesser von 5 $\mu$m bis 8 $\mu$m und einen Hauptachsendurchmesser von 40 $\mu$m oder mehr bei einer Lochdichte von 40 Löcher/mm$^2$ bis 2000 Löcher/mm$^2$ aufweisen,
**dadurch gekennzeichnet, dass**
das Verhältnis (w/z) zwischen dem Hauptachsendurchmesser w ($\mu$m) und einem Spalt z ($\mu$m) zwischen den Löchern in der Nebenachsendurchmesserrichtung 7,0 bis 130 ist.

10. Aufnahmeeinrichtung für eine seltene Zelle zum Aufnehmen einer seltenen Zelle, die in einer Probe enthalten ist, die Einrichtung umfassend:

einen Einlass;
einen Auslass;
einen Kanal, mit dem der Einlass und der Auslass in Kommunikation sind; und
einen Isolierungsabschnitt,
wobei der Isolierungsabschnitt bei einer Position entsprechend einem Abschnitt des Kanals angeordnet ist und das Filter nach Anspruch 9 und einen Halteabschnitt zum Halten des Filters beinhaltet.

11. Verwendung eines Filters zur Isolierung, Detektion oder Analyse einer seltenen Zelle in einer Blutprobe, wobei das Filter wie in Anspruch 9 definiert ist.

12. Kit oder Vorrichtung zur Isolierung oder Detektion einer seltenen Zelle, das oder die lösbar mit einem Strömungskanal verbunden ist, umfassend ein Filter nach Anspruch 9 und einen Filterhalteabschnitt.

## Revendications

1. Procédé pour isoler ou détecter une cellule rare, comprenant filtrer un échantillon sanguin en utilisant un filtre pour isoler ou détecter une cellule rare dans l'échantillon sanguin,
dans lequel le filtre inclut des trous ayant un diamètre de petit axe de 5 $\mu$m à 8 $\mu$m et un diamètre de grand axe de 40 $\mu$m ou plus à une densité de trous de 40 trous/mm$^2$ à 2000 trous/mm$^2$,
**caractérisé en ce que**,
le rapport (w/z) entre le diamètre de grand axe w ($\mu$m) et un espace z ($\mu$m) entre les trous dans la direction du diamètre de petit axe étant de 7,0 à 130.

2. Procédé selon la revendication 1, dans lequel l'échantillon sanguin est filtré de sorte que la capacité de traitement par zone de trous du filtre est de 0,1 nl/$\mu$m$^2$ à 3 nl/$\mu$m$^2$.

3. Procédé selon la revendication 1 ou 2, dans lequel le filtre présente un taux d'ouverture de 10 % à 60 %.

4. Procédé selon l'une quelconque des revendications 1 à 3, dans lequel la pression de filtration durant le filtrage, qui est la différence de pressions dans le système entier depuis l'entrée du système jusqu'à la sortie du système, est de 0,1 kPa à 2,6 kPa.

5. Procédé selon l'une quelconque des revendications 1 à 4, dans lequel la différence entre des pressions sur la surface supérieure et la surface inférieure du filtre durant le filtrage est de 100 Pa ou moins.

6. Procédé selon l'une quelconque des revendications 1 à 5, comprenant l'alimentation de l'échantillon sanguin vers le filtre à un débit moyen de 1 mm/min à 600 mm/min par trou du filtre.

7. Procédé selon l'une quelconque des revendications 1 à 6, dans lequel la cellule rare est une cellule sélectionnée parmi le groupe consistant en une cellule cancéreuse, une cellule tumorale circulante, une cellule endothéliale vasculaire, une cellule précurseur endothéliale vasculaire, une cellule souche cancéreuse, une cellule épithéliale, une cellule souche hématopoïétique, une cellule souche mésenchymateuse, une cellule fœtale, et des combinaisons de celles-ci.

8. Procédé pour analyser une cellule rare dans un échantillon sanguin, comprenant l'analyse d'une cellule rare avec un procédé incluant observer la cinétique de la cellule ou le mesure d'une activité de la cellule, ou avec une analyse génétique de la cellule après que la cellule rare est isolée ou détectée avec le procédé selon l'une quelconque des revendications 1 à 7.

9. Filtre adapté pour une utilisation dans le procédé selon l'une quelconque des revendications 1 à 7, le filtre incluant des trous ayant un diamètre de petit axe de 5 $\mu$m à 8 $\mu$m et un diamètre de grand axe de 40 $\mu$m ou plus à une densité de trous de 40 trous/mm$^2$ à 2000 trous/mm$^2$,
**caractérisé en ce que**,
le rapport (w/z) entre le diamètre de grand axe w ($\mu$m) et un espace z ($\mu$m) entre les trous dans la direction du diamètre de petit axe étant de 7,0 à 130.

10. Appareil de capture de cellule rare pour capturer une cellule rare contenue dans un échantillon, l'appareil comprenant :

une entrée ;
une sortie ;
un canal avec lequel l'entrée et la sortie sont en communication ; et
une partie d'isolation,
dans lequel la partie d'isolation est agencée à une position correspondant à une partie du canal, et inclut le filtre selon la revendication 9 et une partie de maintien pour maintenir le filtre.

11. Utilisation d'un filtre pour isoler, détecter ou analyser une cellule rare dans un échantillon sanguin, dans laquelle ledit filtre est tel que défini dans la revendication 9.

12. Kit ou dispositif pour isoler ou détecter une cellule rare relié de manière amovible à un canal d'écoulement comprenant un filtre selon la revendication 9 et une partie de maintien de filtre.

FIG. 1

ΔP1
(difference between
pressures at entrance
and exit of system)

ΔP2
(difference between pressures on upper side
and lower side of filter)

ΔP2

FIG. 2

FIG. 3

FIG. 4

FIG. 5

FIG. 6

SNU-1/0.4kpa/Blood 8ml/φ5mm

#2          #9          #10

FIG. 7A

SNU-1/1.3kpa/Blood 8ml/φ5mm

#2          #9          #10

FIG. 7B

FIG. 8

FIG. 9

34

FIG. 10

FIG. 11

$$y=0.9435_x+16.266$$
$$R^2=0.9938$$

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2013017429 A
- JP 2010530234 A
- JP 5141919 B
- JP 2013042689 A
- JP 2011163830 A
- US 2012129252 A
- US 2012178097 A
- WO 2014142754 A

### Non-patent literature cited in the description

- **VONA G ; SABILE A ; LOUHA M ; SITRUK V ; ROMANA S ; SCHUTZE K ; CAPRON F ; FRANCO D ; PAZZAGLI M ; VEKEMANS M.** Isolation by Size of Epithelial Tumor Cells: A New Method for the Immunomorphological and Molecular Characterization of Circulating Tumor Cells. *Am J Pathol.,* January 2000, vol. 156 (1), 57-63
- **COUMANS FA ; VAN DALUM G ; BECK M ; TERSTAPPEN LW.** *Filter Characteristics Influencing Circulating Tumor Cell Enrichment from Whole Blood,* April 2013, vol. 8 (4), e61770
- **PARK S ; ANG RR ; DUFFY SP ; BAZOV J ; CHI KN ; BLACK PC ; MA H.** Morphological differences between circulating tumor cells from prostate cancer patients and cultured prostate cancer cells. *PLOSONE,* January 2014, vol. 9 (1), e85264
- **XU W ; MEZENCEV R ; KIM B ; WANG L ; MCDONALD J ; SULCHEK T.** *Cell Stiffness Is a Biomarker of the Metastatic Potential of Ovarian Cancer Cells,* October 2012, vol. 7 (10), e46609
- **ZHANG W ; KAI K ; CHOI DS ; IWAMOTO T ; NGUYEN YH ; WONG H ; LANDIS MD ; UENO NT ; CHANG J ; QIN L.** *Microfluidics separation reveals the stem-cell-like deformability of tumor-initiating cells,* 2012
- **R. NEGISHI et al.** Development of the automated circulating tumor cell recovery system with microcavity array. *Biosensors and Bioelectronics,* 2014